# EUROPEAN PATENT APPLICATION

(11) **EP 0 696 588 A1**
(43) Date of publication of application: **14.02.1996**
(21) Application number: 95117397.0
(22) Date of filing: 02.07.1993
(51) Int. Cl.: C07D 401/04, C07D 407/10, C07D 407/04, C07D 409/04, C07D 495/04, A01N 43/54, C07D 239/36, C07D 239/34, C07D 239/91

(54) **2-Arylpyrimidines and herbicidal use thereof**

(30) Priority: 17.07.1992 US 916247; 17.07.1992 US 916780; 20.05.1993 US 62802
(62) Divisional of application: 93305207.8
(71) Applicant: ROHM AND HAAS COMPANY, Philadelphia, Pennsylvania 19106-2399 (US)
(72) Inventor: Tice, Colin M., Melrose Park, Pennsylvania 19126 (US)
(74) Representative: Tanner, James Percival

(57) **Abstract**

A class of 2-arylpyrimidines which is useful in the control of weeds is of the general formula: 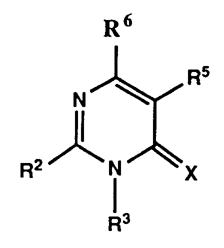
wherein R² is an optionally substituted aromatic ring; R³ is a saturated or unsaturated alkyl group; R⁵ and R⁶ are joined together to form a ring; and X is oxygen or sulfur.

## Description

A need continues for novel and improved herbicidal compounds and compositions. This is particularly so since the targets of herbicides can become resistant to known herbicides over time and after use of such compositions. Additionally, economic and environmental considerations can favour herbicides having different modes of performance than those currently used. This invention relates to novel 2-arylpyrimidines and their use as broad spectrum herbicides.

Accordingly in one aspect the present invention provides compounds of the formula: 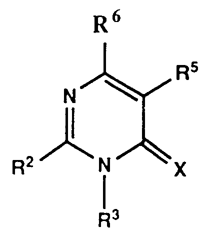
wherein
(a) R² is a furyl, phenyl, naphthyl, pyridyl, or thienyl group,
   each of said group is optionally substituted with up to three substituents independently selected from a bromo, chloro, fluoro, (C₁-C₁₂)alkyl, cyclo(C₃-C₈)alkyl, (C₂-C₁₂)alkenyl, cyclo(C₃-C₈)alkenyl, (C₂-C₁₂)alkynyl, halo(C₁-C₁₂)alkyl, polyhalo(C₁-C₁₂)alkyl, halo(C₂-C₁₂)alkenyl, polyhalo(C₂-C₁₂)alkenyl, halo(C₂-C₆)alkynyl, polyhalo(C₂-C₆)alkynyl, (C₁-C₁₂)alkoxy, (C₁-C₁₂)alkylthio, (C₁-C₁₂)alkylsulfonyl, (C₁-C₁₂)alkylsulfinyl, phenyl, phen(C₁-C₁₂)alkyl, phen(C₂-C₁₂)alkenyl, phen(C₂-C₁₂)alkynyl, cyano, halo(C₁-C₁₂)alkoxy, carbo(C₁-C₆)alkoxy, 1,3-dioxolan-2-yl, hydroxyimino, or nitro group; and when R² is pyridyl, such pyridyl group is optionally substituted with oxygen on the nitrogen of the pyridyl group; or R² is a furyl, phenyl, naphthyl, pyridyl or thienyl group having a fused ring moiety composed of an oxymethyleneoxy or an oxoethyleneoxy link bonds to adjacent carbon atoms or said group;
(b) R³ is a (C₁-C₃)alkyl, halo(C₁-C₃)alkyl, polyhalo(C₁-C₃)alkyl, (C₃-C₄)alkenyl, (C₅-C₆)alkenynyl, (C₃-C₆)alkynyl, (C₁-C₆)alkoxy(C₁-C₆)alkyl, di(C₁-C₆)alkoxy(C₁-C₆)alkyl, halo(C₁-C₆)alkoxy(C₁-C₆)alkyl, or 2-oxo(C₂-C₃)alkyl, trimethylsilyl(C₃-C₄)alkynyl or cyano(C₁-C₆)alkyl group, each of said (C₃-C₄)alkenyl, or (C₃-C₆)alkynyl group is optionally substituted with up to five halogens; and
(c) R⁵ is a hydrogen, (C₁-C₅)alkyl, (C₃-C₆)alkenyl, (C₂-C₆)alkynyl, halo(C₁-C₆)alkyl, polyhalo(C₁-C₆)alkyl, halo(C₂-C₆)alkenyl, polyhalo(C₂-C₆)alkenyl, halo(C₂-C₆)alkynyl, polyhalo(C₂-C₆)alkynyl, halo(C₁-C₆)alkoxy, polyhalo(C₁-C₆)alkoxy, trimethylsilyl(C₂-C₃)alkynyl, (C₁-C₆)alkoxy, (C₁-C₆)alkylthio, (C₁-C₃)alkoxycarbonyl(C₁-C₃)alkyl, halo, or cyano group; and
(d) R⁶ is a hydrogen, halo, (C₁-C₈)alkyl, (C₂-C₆)alkenyl, (C₂-C₆)alkynyl, halo(C₁-C₆)alkyl, polyhalo(C₁-C₆)alkyl, halo(C₂-C₆)alkenyl, polyhalo(C₂-C₆)alkenyl, halo(C₂-C₆)alkynyl, polyhalo(C₂-C₆)alkynyl, (C₁-C₆)alkoxy, (C₁-C₆)alkoxy(C₁-C₄)alkyl, (C₁-C₆)alkylthio, (C₁-C₃)alkoxycarbonyl, (C₁-C₃)alkoxycarbonyl(C₁-C₃)alkyl, (C₆-C₁₀)aryl, ar(C₁-C₄)alkyl, cyclo(C₃-C₇)alkyl, (C₄-C₅)heterocyclyl selected from a group consisting of furyl, pyridyl and thienyl, (C₁-C₃)alkylamino, di(C₁-C₃)alkylamino, di(C₁-C₃)alkylaminocarbonyl, halo(C₁-C₆)alkylthio, polyhalo(C₁-C₆)alkythio, halo(C₁-C₆)alkoxy, polyhalo(C₁-C₆)alkoxy, (C₆-C₁₀)aryloxy or cyano group; said (C₆-C₁₀)aryl, ar(C₁-C₄)alkyl and (C₆-C₁₀)aryloxy groups being optionally substituted with up to three substituents independently selected from bromo; chloro; fluoro; (C₁-C₁₂)alkyl, cyclo(C₃-C₈)alkyl, (C₂-C₁₂)alkenyl, cyclo(C₃-C₈)alkenyl, (C₂-C₁₂)alkynyl, halo(C₁-C₁₂)alkyl, polyhalo(C₁-C₁₂)alkyl, halo(C₂-C₁₂)alkenyl, polyhalo(C₂-C₁₂)alkenyl, halo(C₂-C₆)alkynyl, polyhalo(C₂-C₆)alkynyl; (C₁-C₁₂)alkoxy, (C₁-C₁₂)alkylthio, (C₁-C₁₂)alkylsulfonyl, (C₁-C₁₂)alkylsulfinyl, phenyl, phen(C₁-C₁₂)alkyl, phen(C₂-C₁₂)alkenyl, phen(C₂-C₁₂)alkynyl, cyano, halo(C₁-C₁₂)alkoxy, 1,3-dioxalan-2-yl, hydroxyimino, and nitro;
   or alternatively R⁵ and R⁶ are joined together to form a fused ring moiety bonded to the pyrimidine ring at the 5 and 6 positions and containing two to five atoms in its link, each of said atoms being independently carbon, oxygen or sulfur of which the carbon atoms are optionally substituted with (C₁-C₃)alkyl, polyhalo(C₁-C₃)alkyl or halo groups; and
(e) X is oxygen or sulfur.

It is to be understood that the prefix term "halo" designates a halogen substituent (such as fluorine, chlorine, bromine, or iodine) and that "polyhalo" designates two or more substituents independently selected halogens. It is further to be understood that, unless otherwise specified, use of the prefix "halo" without a concurrent use of the prefix "polyhalo" is not intended to limit the invention to singularly halogenated compounds. This invention also teaches methods of preparing these compounds as well as methods of using the compounds as herbicides.

More specifically, the present invention comprises compounds of the general formula: 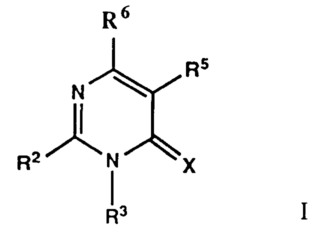
wherein
R² is an aryl or heteroaromatic group, preferably furyl, phenyl, naphthyl, pyridyl, or thienyl, and may be optionally substituted with up to three substituents independently selected from bromo; chloro; fluoro; (C₁-C₁₂)alkyl, preferably(C₁-C₆)alkyl; cyclo(C₃-C₈)alkyl, preferably cyclo(C₅-C₆)alkyl; (C₂-C₁₂)alkenyl, preferably (C₂-C₆)alkenyl; cyclo(C₃-C₈)alkenyl; (C₂-C₁₂)alkynyl, preferably (C₂-C₆)alkynyl; halo(C₁-C₁₂)alkyl, preferably halo(C₁-C₆)alkyl; polyhalo(C₁-C₁₂)alkyl, preferably polyhalo(C₁-C₆)alkyl; halo(C₂-C₁₂)alkenyl, preferably halo(C₂-C₆)alkenyl; polyhalo(C₂-C₁₂)alkenyl, preferably polyhalo(C₂-C₆)alkenyl; halo(C₂-C₆)alkynyl; polyhalo(C₂-C₆)alkynyl; (C₁-C₁₂)alkoxy, preferably (C₁-C₆)alkoxy; (C₁-C₁₂)alkylthio, preferably (C₁-C₆)alkylthio; (C₁-C₁₂)alkylsulfonyl; (C₁-C₁₂)alkylsulfinyl; phenyl; phen(C₁-C₁₂)alkyl; phen(C₂-C₁₂)alkenyl; phen(C₂-C₁₂)alkynyl; cyano; halo(C₁-C₁₂)alkoxy, preferably halo(C₁-C₆)alkoxy; 1,3-dioxalan-2-yl; hydroxyimino, polyhalo(C₁-C₁₂)alkoxy; and nitro. Substituent groups can be branched or unbranched. Preferred phenyl groups are phenyl, 3-methylphenyl, 3-methoxyphenyl, 3-nitrophenyl, 4-fluorophenyl, 4-chlorophenyl, 3-trifluoromethylphenyl, 3-bromophenyl, 3-chlorophenyl, 3-fluorophenyl, 3-trifluoromethoxyphenyl, 3-cyanophenyl, 3-(1,3-dioxolan-2-yl)phenyl, 3-(hydroxyimino)phenyl, 2-fluorophenyl, 2-chlorophenyl, 2-trifluoromethoxyphenyl, 3,5-difluorophenyl, 3,5-dichlorophenyl, 2,4-difluorophenyl, 2,5-difluorophenyl, 3-chloro-4-fluorophenyl, 3,4-difluorophenyl, 3-fluoro-5-trifluoromethylphenyl, 3,4,5-trifluorophenyl; more preferably phenyl, 3-fluorophenyl, and 3-chlorophenyl. Preferred pyridyl groups are 6-chloro-2-pyridyl; 3-pyridyl; 1-methyl-3-pyridinium; 5-bromo-3-pyridyl; 5,6-dichloro-3-pyridyl; 5-chloro-3-pyridyl, 1-oxo-3-pyridyl; 4-pyridyl; 2-fluoro-4-pyridyl; 2-chloro-4-pyridyl; 2-chloro-6-methyl-4-pyridyl; 2-methyl-4-pyridyl, 2-methoxy-4-pyridyl, 2-cyano-4-pyridyl,1-oxo-4-pyridyl, 2,6-difluoro-4-pyridyl and 2,6-dichlor-4-pyridyl. More preferred are 2-chloro-4-pyridyl; 2-fluoro-4-pyridyl; and 2,6-dichloro-4-pyridyl. The pyridyl groups can also be present as a salt, such as 1-methyl-3-pyridinium iodide or 3-pyridinium hydrochloride. Preferred furyl groups are 2-furyl and 3-furyl. A preferred naphthyl group is 2-naphthyl. Preferred thienyl groups are 2-thienyl, 3-thienyl, 4-chloro-2-thienyl, 5-chloro-2-thienyl, 5-chloro-3-thienyl and 2,5-dichloro-3-thienyl.

In the case of R² being a pyridyl group, an additional selection of substituent groups is oxygen substituted on the nitrogen atom of the pyridyl ring; e.g. N-oxo groups, such as 1-oxo-3-pyridyl or 1-oxo-4-pyridyl. Optionally, each of the furyl, phenyl, naphthyl, pyridyl and thienyl groups can have a fused ring moiety such that the fused ring is composed of alkylenedioxy, e.g. an oxymethyleneoxy (-O-CH₂-O-) link or an oxyethyleneoxy (-O-CH₂CH₂-O-) link which is bonded to adjacent carbon atoms of the group. For example, 3,4-methylenedioxyphenyl.

R³ is an alkyl, alkenyl, alkynyl, alkenynyl, alkoxyalkyl, dialkoxyalkyl, haloalkoxyalkyl, oxoalkyl, trimethylsilylalkynyl, cyanoalkyl or aryl group. Preferably, R³ is a (C₁-C₃)alkyl; (C₃-C₄)alkenyl; or (C₃-C₆)alkynyl group, each of which may be optionally substituted with up to five halogens; or a (C₁-C₆)alkoxy(C₁-C₆)alkyl, di(C₁-C₆)alkoxy(C₁-C₆)alkyl, halo(C₁-C₆)alkoxy(C₁-C₆)alkyl, 2-oxo(C₂-C₃)alkyltrimethylsilyl (C₃-C₄)alkynyl or cyano(C₁-C₆)alkyl group. A preferred (C₁-C₃)alkyl group is ethyl. Preferred alkenyl and halogen substituted alkenyl groups are (C₃-C₄)alkenyls, such as allyl and 3-chloroallyl. Preferred alkynyl groups are (C₃-C₆)alkynyl, such as pentynyl, propynyl and butynyl, more preferably pent-2-ynyl, prop-2-ynyl, and but-2-ynyl. Preferred halogen substituted (C₃-C₆)alkynyl groups are iodopropargyl and bromopropargyl. Preferred (C₁-C₆)alkoxy(C₁-C₆)alkyls are (C₁-C₂)alkoxy(C₁-C₃)alkyl, more preferably methoxymethyl and 2-methoxyethyl, and most preferably methoxymethyl. Preferred di(C₁-C₆)alkoxy(C₁-C₆)alkyls are di(C₁-C₂)alkoxy(C₁-C₃)alkyls, more preferably 2,2-dimethoxypropyl. A preferred 2-oxo(C₂-C₃)alkyl is acetonyl. A preferred trimethylsilyl (C₃-C₄)alkynyl is 3-(trimethylsilyl)propargyl. A preferred cyano (C₁-C₆)alkyl is cyanomethyl. Preferred alkenynyls are (C₅-C₆)alkenynyls, more preferably pent-4-en-2-ynyl.

R⁵ is a hydrogen, halo, alkyl, alkenyl, alkynyl, alkoxy, alkylthio, alkoxycarbonylalkyl, haloalkyl, haloalkenyl, haloalkynyl, haloalkoxy, polyhaloalkyl, polyhaloalkenyl, polyhaloalkynyl, polyhaloalkoxy, trimethylsilylalkynyl or cyano group. Preferred R⁵ substituents are hydrogen, (C₁-C₅)alkyl, (C₃-C₆)alkenyl, (C₂-C₆)alkynyl, trimethylsilyl(C₂-C₃)alkynyl, (C₁-C₆)alkoxy, halo- or polyhalo(C₁-C₆)alkyl, halo- or polyhalo(C₂-C₆)alkenyl, halo- or polyhalo(C₂-C₆)alkynyl, halo (C₁-C₆)alkoxy, polyhalo (C₁-C₆)alkoxy, (C₁-C₃)alkoxycarbonyl(C₁-C₃)alkyl, (C₁-C₆)alkylthio, halo and cyano. Preferred (C₁-C₅)alkyls are methyl, ethyl, n-propyl and iso-propyl, more preferably methyl and ethyl. Preferred (C₂-C₆)alkynyls are (C₂-C₄)alkynyls, more preferably prop-2-ynyl. Preferred (C₁-C₆)alkoxys are (C₁-C₂)alkoxys, more preferably methoxy. Preferred (C₁-C₆)alkylthios are (C₁-C₂)alkylthios, more preferably methylthio. A preferred alkoxycarbonyalkyl is methoxycarbonylmethyl. Preferred (C₃-C₆)alkenyls are (C₃-C₄)alkenyl, more preferably allyl. Preferred halo(C₁-C₆)alkyls and polyhalo(C₁-C₆)alkyls are halo(C₁-C₂)alkyls and polyhalo(C₁-C₂)alkyls, more preferably fluoromethyl and trifluoromethyl. Preferred halo(C₁-C₆)alkoxys and polyhalo(C₁-C₆)alkoxys are halo(C₁-C₂)alkoxys, and polyhalo(C₁-C₂)alkoxys more preferably difluoromethoxy and trifluoromethoxy. Preferred halos are chloro and fluoro. A preferred trimethylsilyl(C₂-C₃)alkynyl is trimethylsilylethynyl.

R⁶ is a hydrogen, halo, alkyl, alkenyl, alkynyl, alkoxy, alkylthio, alkoxyalkyl, alkoxycarbonyl, alkoxycarbonylalkyl, haloalkyl, polyhaloalkyl, cycloalkyl, haloalkylthio, haloalkenyl, polyhaloalkenyl, haloalkynyl, polyhaloalkynyl, haloalkoxy, polyhaloalkoxy, polyhaloalkylthio, aryl, aryloxy, heterocyclyl selected from furyl, pyridyl and thienyl, aralkyl, alkylamino, dialkylamino, dialkylaminocarbonyl, or cyano group. Preferred R⁶ are hydrogen, halo, straight (C₁-C₈)alkyl, branched (C₃-C₈)alkyl, (C₂-C₆)alkenyl, (C₂-C₆)alkynyl, halo(C₁-C₆)alkyl or polyhalo(C₁-C₆)alkyl, halo(C₂-C₆)alkenyl or polyhalo(C₂-C₆)alkenyl, halo(C₂-C₆)alkynyl or polyhalo(C₂-C₆)alkynyl, (C₁-C₆)alkoxy(C₁-C₄)alkyl, (C₁-C₆)alkoxy, (C₁-C₆)alkylthio, (C₁-C₃)alkoxycarbonyl, (C₁-C₃)alkoxycarbonyl(C₁-C₃)alkyl, substituted or unsubstituted (C₆-C₁₀)aryl, substituted or unsubstituted (C₆-C₁₀)aryloxys, substituted or unsubstituted ar(C₁-C₄)alkyl, cyclo(C₃-C₇)alkyl, halo(C₁-C₆)alkylthio, polyhalo (C₁-C₆)alkythio, halo(C₁-C₆)alkoxy, polyhalo (C₁-C₆)alkoxy, (C₄-C₅)heterocyclyl, (C₁-C₃)alkylamino, di(C₁-C₃)alkylamino, di(C₁-C₃)alkyl aminocarbonyl, and cyano. The aryl portion of the foregoing (C₆-C₁₀)aryl, (C₆-C₁₀)aryloxy and aryl(C₁-C₄)alkyl groups can be optionally substituted with up to three substituents independently selected from bromo; chloro; fluoro; (C₁-C₁₂)alkyl, preferably (C₁-C₆)alkyl; cyclo(C₃-C₈)alkyl, preferably cyclo(C₅-C₆)alkyl; (C₂-C₁₂)alkenyl, preferably (C₂-C₆)alkenyl; cyclo(C₃-C₈)alkenyl; (C₂-C₁₂)alkynyl, preferably (C₂-C₆)alkynyl; halo(C₁-C₁₂)alkyl, preferably halo(C₁-C₆)alkyl; polyhalo(C₁-C₁₂)alkyl, preferably polyhalo(C₁-C₆)alkyl; halo(C₂-C₁₂)alkenyl, preferably halo(C₂-C₆)alkenyl; polyhalo(C₂-C₁₂)alkenyl, preferably polyhalo(C₂-C₆)alkenyl; halo(C₂-C₆)alkynyl; polyhalo(C₂-C₆)alkynyl; (C₁-C₁₂)alkoxy, preferably (C₁-C₆)alkoxy; (C₁-C₁₂)alkylthio, preferably (C₁-C₆)alkylthio; (C₁-C₁₂)alkylsulfonyl; (C₁-C₁₂)alkylsulfinyl; phenyl; phen(C₁-C₁₂)alkyl; phen(C₂-C₁₂)alkenyl; phen(C₂-C₁₂)alkynyl; cyano; halo(C₁-C₁₂)alkoxy, preferably halo(C₁-C₆)alkoxy; 1,3-dioxalan-2-yl; hydroxyimino; and nitro. Preferred (C₁-C₈)alkyls are straight (C₁-C₇)alkyls and branched (C₃-C₈)alkyls, preferably methyl, ethyl, n-propyl, n-butyl, n-pentyl, n-hexyl, n-heptyl, s-butyl, i-propyl, i-butyl and t-butyl; more preferably methyl, ethyl, n-propyl, s-butyl, i-propyl and t-butyl. A preferred (C₂-C₆)alkenyl is 2-methyl-1-propenyl. A preferred (C₆-C₁₀)aryl is phenyl. A preferred (C₆-C₁₀)aryloxy is phenoxy. Preferred (C₄-C₅)heterocyclyls are 3-thienyl, 3-furyl, 2-thienyl and 4-pyridyl; most preferably 3-thienyl. Preferred (C₁-C₆)alkoxys are (C₁-C₅)alkoxys, more preferably methoxy and ethoxy. A preferred (C₁-C₃)alkoxycarbonyl is ethoxycarbonyl. Preferred (C₂-C₆)alkynyls are but-2-ynyl, but-3-ynyl, and prop-2-ynyl. Preferred halos are fluoro, bromo, and chloro; more preferably chloro and bromo. Preferred halo(C₁-C₆)alkyls and polyhalo(C₁-C₆)alkyls are halo(C₁-C₃)alkyls and polyhalo(C₁-C₃)alkyls, more preferably trifluoromethyl, pentafluoroethyl, trichloromethyl, bromomethyl, chloromethyl, difluoromethyl, and chlorodifluoromethyl; most preferably trifluoromethyl. Preferred halo(C₁-C₆)alkoxys and polyhalo(C₁-C₆)alkoxys are halo(C₁-C₃)alkoxys and polyhalo(C₁-C₃)alkoxys, more preferably difluoromethoxy and trifluoromethoxy. Preferred (C₁-C₆)alkylthios are (C₁-C₅)alkylthios, more preferably methylthio. A preferred (C₁-C₆)alkoxy(C₁-C₄)alkyl is methoxymethyl. A preferred ar(C₁-C₄)alkyl is benzyl. Preferred cyclo(C₃-C₇)alkyls are cyclopropyl, cyclobutyl and cyclopentyl. A preferred di(C₁-C₃)alkylamino is dimethylamino. A preferred di(C₁-C₃)alkylaminocarbonyl is dimethylaminocarbonyl.

Alternatively R⁵ and R⁶ are joined together to form a fused ring moiety bonded to the pyrimidine ring at the 5 and 6 positions containing two to five atoms in its link, each of such atoms independently selected from carbon, oxygen and sulfur. For the purposes herein, - R⁵--- R⁶ - can alternatively be termed the R⁵-R⁶ link. As an illustration of one example of what is intended, when the R⁵-R⁶ link is designated as the four atom link -CH₂CH₂CH₂CH₂-, the following structure is attained: 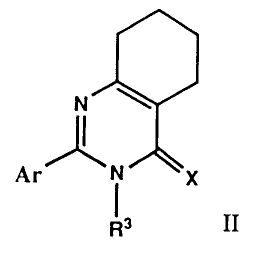
For instance, possible R⁵-R⁶ links are dimethylene (-CH₂CH₂-), trimethylene (-CH₂CH₂CH₂-), tetramethylene (-CH₂CH₂CH₂CH₂-) and pentamethylene (-(CH₂)₅-). Heteroatom substitution in the R⁵-R⁶ link using oxygen or sulfur can be present. Preferably at most only one oxygen or sulfur is used in the R⁵-R⁶ link when a heteroatom substitution is present. Examples are thioethylene (-SCH₂CH₂-) and oxyethylene (-OCH₂CH₂-). The R⁵-R⁶ link need not be a saturated link, but can contain double bonds. Examples are 1,3-butadienylene (-CH=CHCH=CH-) and thiovinylene (-SCH=CH-). The R⁵-R⁶ link can be a substituted link. For example, an ethyl substituent on the R⁵-R⁶ link can be present, such as in 1-ethyl-thiovinylene ( -CH=C(CH₂CH₃ )S-). Accordingly, embodiments of the invention can have (C₁-C₃)alkyl groups, such as methyl, ethyl, and propyl, polyhalo(C₁-C₃)alkyl and halogen atoms as substituents on the R⁵-R⁶ link.

X is oxygen or sulfur, preferably oxygen.

A preferred embodiment of this invention are the compounds represented by formula I wherein X is oxygen and R² is substituted or unsubstituted phenyl, pyridyl, or thienyl.

A more preferred embodiment of this invention are the compounds represented by formula I wherein X is oxygen; R² is substituted or unsubstituted phenyl, substituted or unsubstituted pyridyl or substituted or unsubstituted thienyl; and R³ is (C₃-C₆)alkynyl.

A still more preferred embodiment of this invention is the compound represented by formula I wherein X is oxygen; R² is substituted or unsubstituted phenyl, substituted or unsubstituted pyridyl or substituted or unsubstituted thienyl; R³ is (C₃-C₆)alkynyl; and R⁵ and R⁶ are independently selected from hydrogen, halo, (C₁-C₄)alkyl, polyhalo(C₁-C₄)alkyl, and (C₁-C₄)alkoxy. R⁶ can be also unsubstituted or substituted phenyl.

Even more preferred is the compound represented by formula I wherein X is oxygen; R² is phenyl, 3-substituted phenyl (i.e. meta-substituted phenyl), 3,5-disubstituted-phenyl or 3,4,5-trisubstituted phenyl, 2-substituted-4-pyridyl or 2,6-disubstituted-4-pyridyl or 3-thienyl or 5-substituted-3-thienyl; R³ is (C₃-C₆)alkynyl; and R⁵ and R⁶ are independently selected from hydrogen, halo, (C₁-C₄)alkyl, polyhalo(C₁-C₄)alkyl and (C₁-C₄)alkoxy. R⁶ can be also unsubstituted or substituted phenyl.

A yet more preferred embodiment of this invention is the compound represented by formula I wherein X is oxygen; R² is phenyl, 3-fluorophenyl, 3-chlorophenyl, 3,5-difluorophenyl, 3,5-dichlorophenyl, 3,4,5-trifluorophenyl, 2-chloro-4-pyridyl, 2-fluoro-4-pyridyl, 2,6-dichloro-4-pyridyl or 3-thienyl or 5-chloro-3-thienyl; R³ is propargyl; R⁵ is hydrogen, methyl, ethyl, methoxy, fluoro or chloro; and R⁶ is methyl, ethyl, isopropyl, n-propyl, n-butyl, s-butyl, i-butyl, t-butyl, trifluoromethyl, difluoromethyl, phenyl, chloro, bromo, or fluoro.

An additionally preferred embodiment of this invention is the compound represented by formula I wherein X is oxygen; R² is substituted or unsubstituted phenyl or substituted or unsubstituted pyridyl; R³ is (C₃-C₆) alkynyl; and R⁵ and R⁶ are joined to form a link comprising two, three or four carbons, and zero or one oxygen or sulfur atoms. When R² is substituted, the substituents described hereinabove are used.

Even more preferred is the compound represented by formula I wherein X is oxygen; R² is phenyl, 3-substituted phenyl (i.e. meta-substituted phenyl), 3,5-disubstituted-phenyl or 2-substituted-4-pyridyl or 2,6-disubstituted-4-pyridyl; R³ is propargyl; and the -R⁵-R⁶ - link is -CH=CH-CH=CH-, or -S-CH=CH-, or -(CH₂)_{y}- wherein y is 3 or 4. When R² is substituted, the substituents described hereinabove are used.

A yet more preferred embodiment of this invention is the compound represented by formula I wherein X is oxygen; R² is phenyl, 3-fluorophenyl, 3-chlorophenyl, 3,5-difluorophenyl, 3,5-dichlorophenyl, 2-chloro-4-pyridyl, 2-fluoro-4-pyridyl, or 2,6-dichloro-4-pyridyl; R³ is propargyl; and the -R⁵-R⁶- link is -(CH₂)_{y}-wherein y is 3 or 4.

Preferred compounds include
(a) 5,6-diethyl-2-phenyl-3-propargyl-4(3H)-pyrimidinone;
(b) 5-ethyl-2-phenyl-3-propargyl-6-trifluoromethyl-4(3H)-pyrimidin one;
(c) 5-ethyl-6-(1-methylethyl)-2-phenyl-3-propargyl-4(3H)-pyrimidin one;
(d) 6-chloro-5-ethyl-2-phenyl-3-propargyl-4(3H)-pyrimidinone;
(e) 5,6-diethyl-2-(3-fluorophenyl)-3-propargyl-4(3H)-pyrimidinone;
(f) 2-(2,6-dichloro-4-pyridyl)-5,6-diethyl-3-propargyl-4(3H)-pyrimidi none;
(g) 5,6-diethyl-2-(3,5-difluorophenyl)-3-propargyl-4(3H)-pyrimidinone;
(h) 5-ethyl-2-phenyl-3-propargyl-6-propyl-4(3H)-pyrimidinone.
(i) 6-difluoromethyl-5-ethyl-2-phenyl-3-propargyl-4(3H)-pyrimidin one;
(j) 6-ethyl-5-methoxy-2-phenyl-3-propargyl-4(3H)- pyrimidinone;
(k) 2-phenyl-3-propargyl-3,5,6,7-tetrahydro-4H-cyclopentapyrimidin-4-one; or
(l) 2-phenyl-3-propargyl-3,5,6,7,8-pentahydro-4H-cyclohexapyrimidi n-4-one.

In a further aspect the invention provides a herbicidal composition comprising a herbicidally effective amount of a compound as defined above, and an agronomically acceptable carrier. Another aspect of the invention is a method of controlling a weed comprising applying a herbicidally effective amount of a composition or compound as defined above to said weed or to the locus of said weed or to the growth medium of said weed. The invention also comprises in a still further aspect the use of a compound or composition as defined above as a herbicide.

Compounds encompassed by the present invention include, but are not limited to, those illustrated in Table 1. The synthesis methods (i.e., "A", "B" etc.) specified in the table are described hereinafter in this specification. The sequence of letters in the "Synthesis" column indicated the relative sequence of steps performed. For instance, "D + A" indicates the steps of procedure D were first performed, followed by the steps of Procedure A.

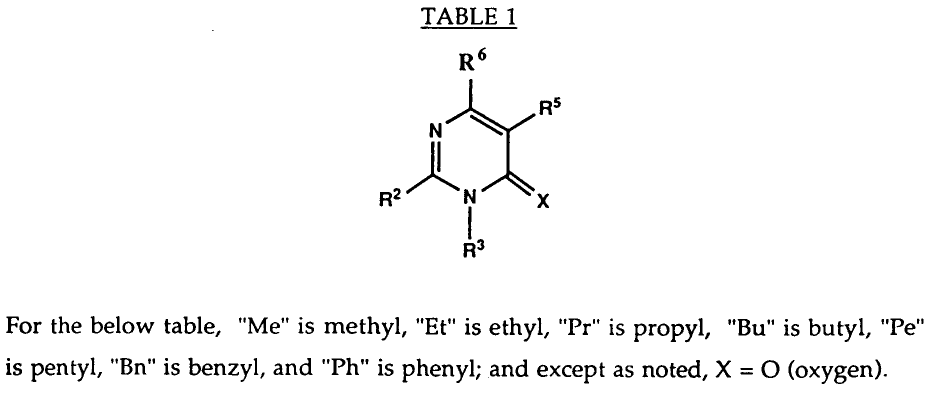

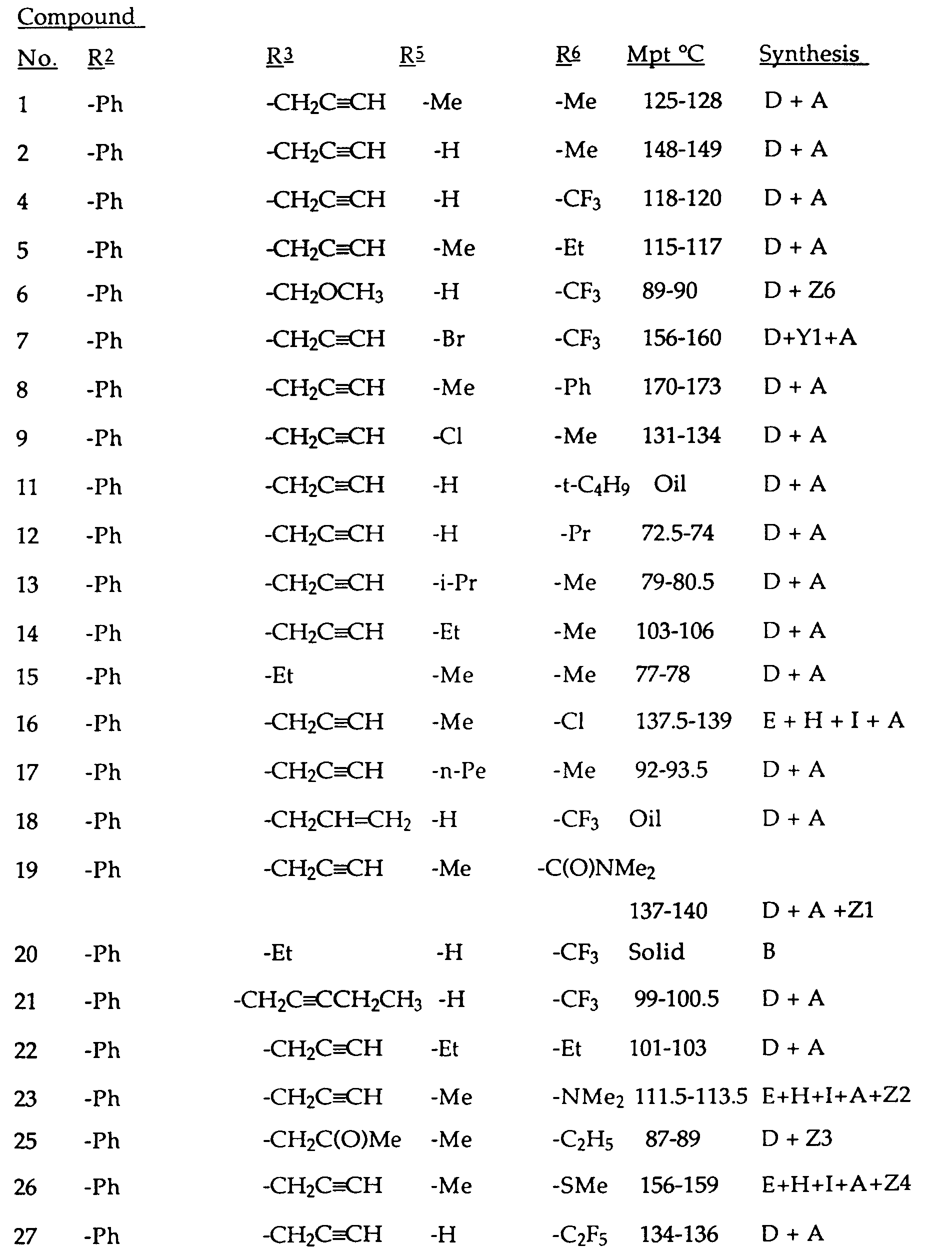

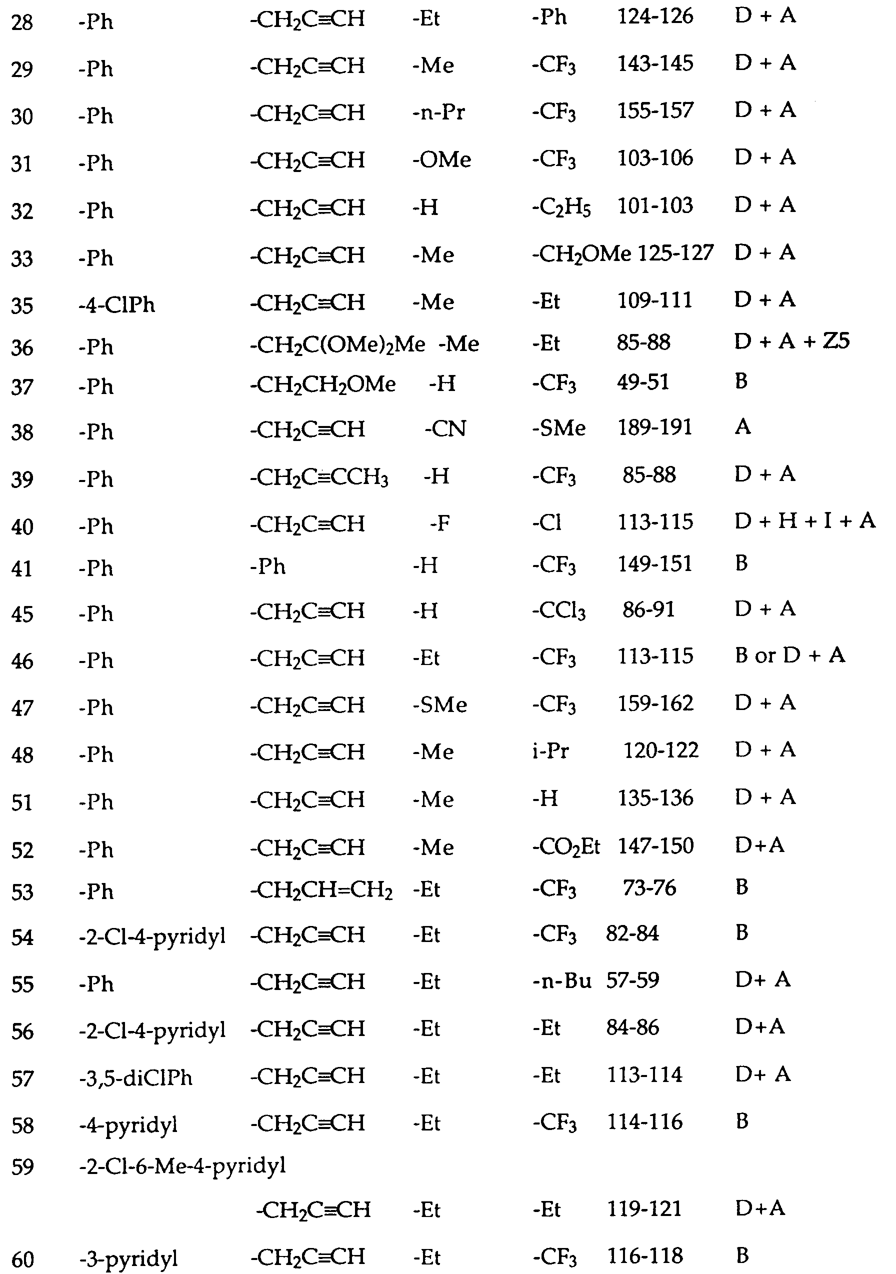

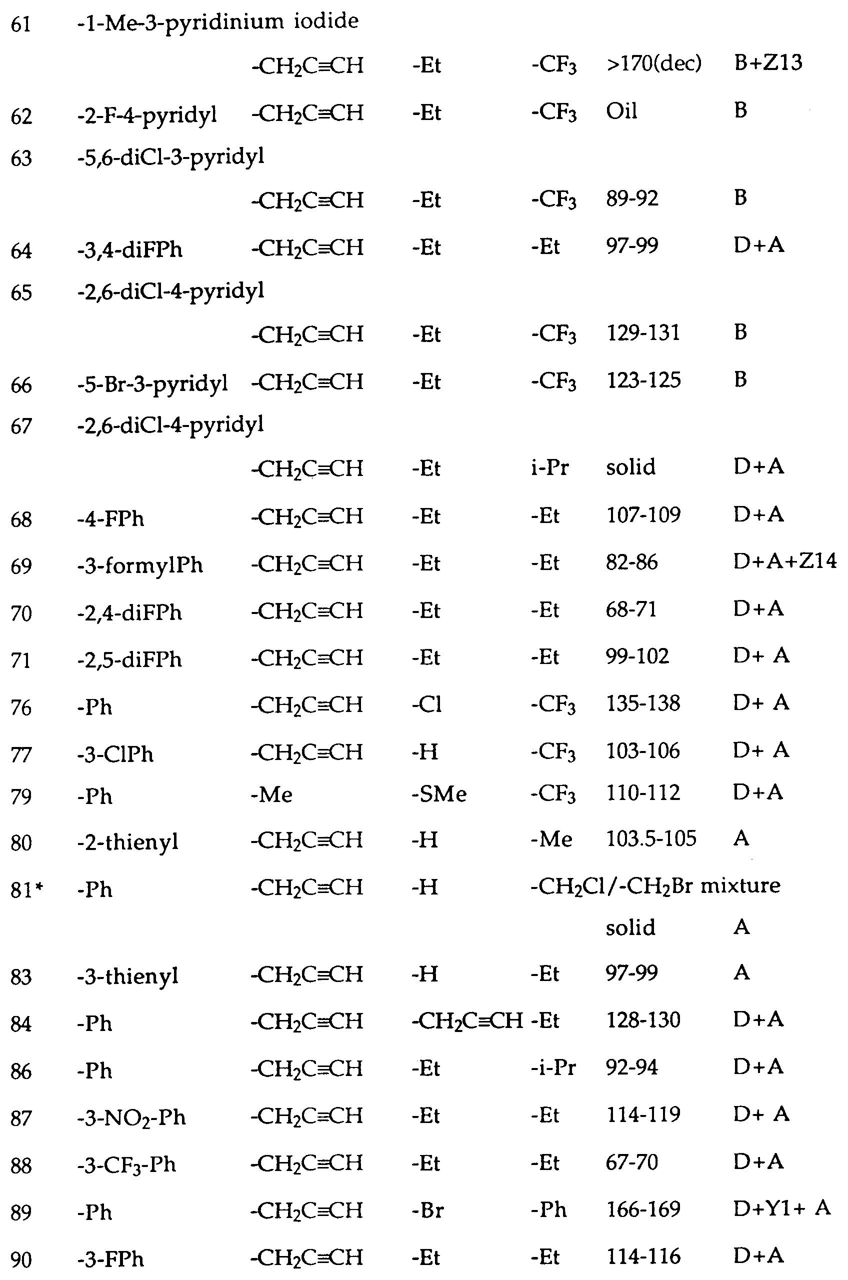

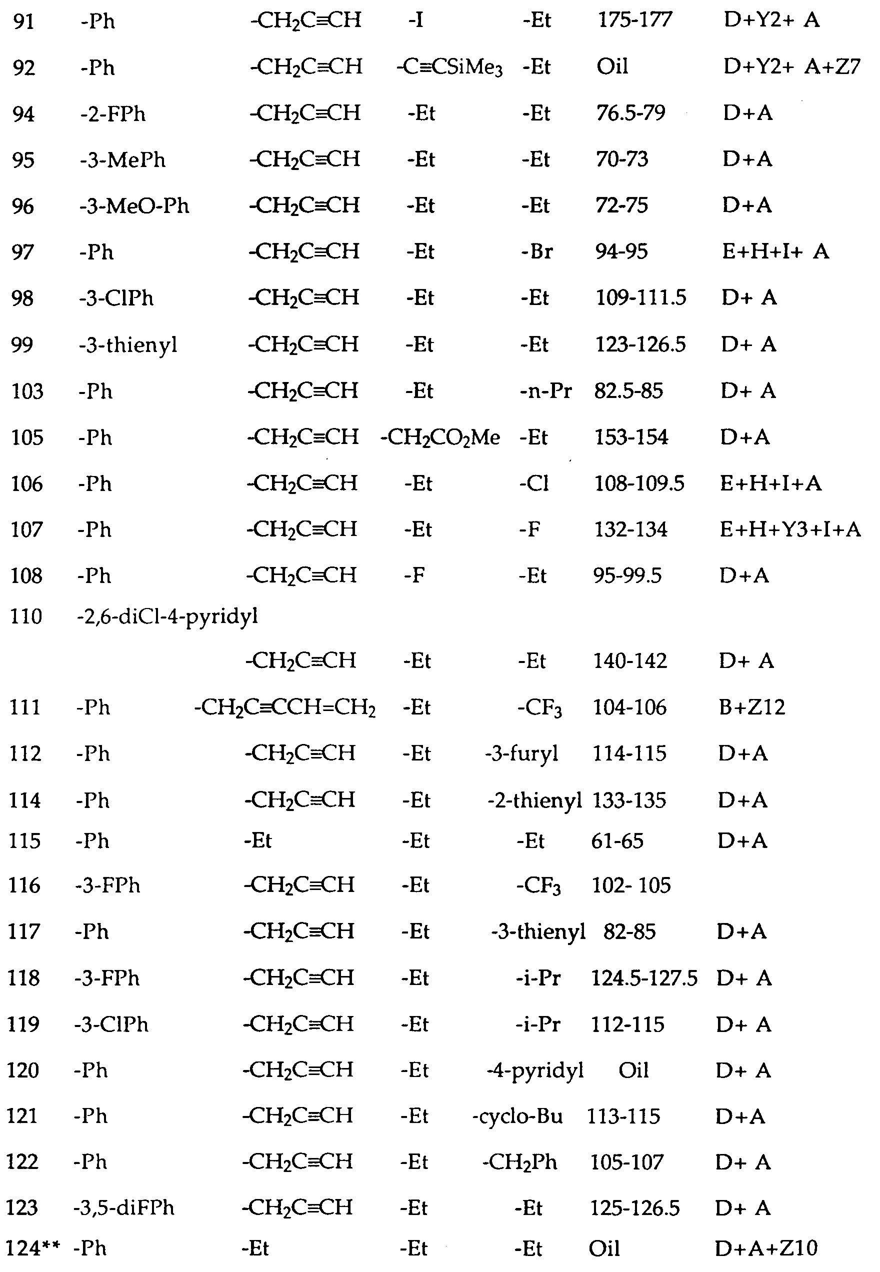

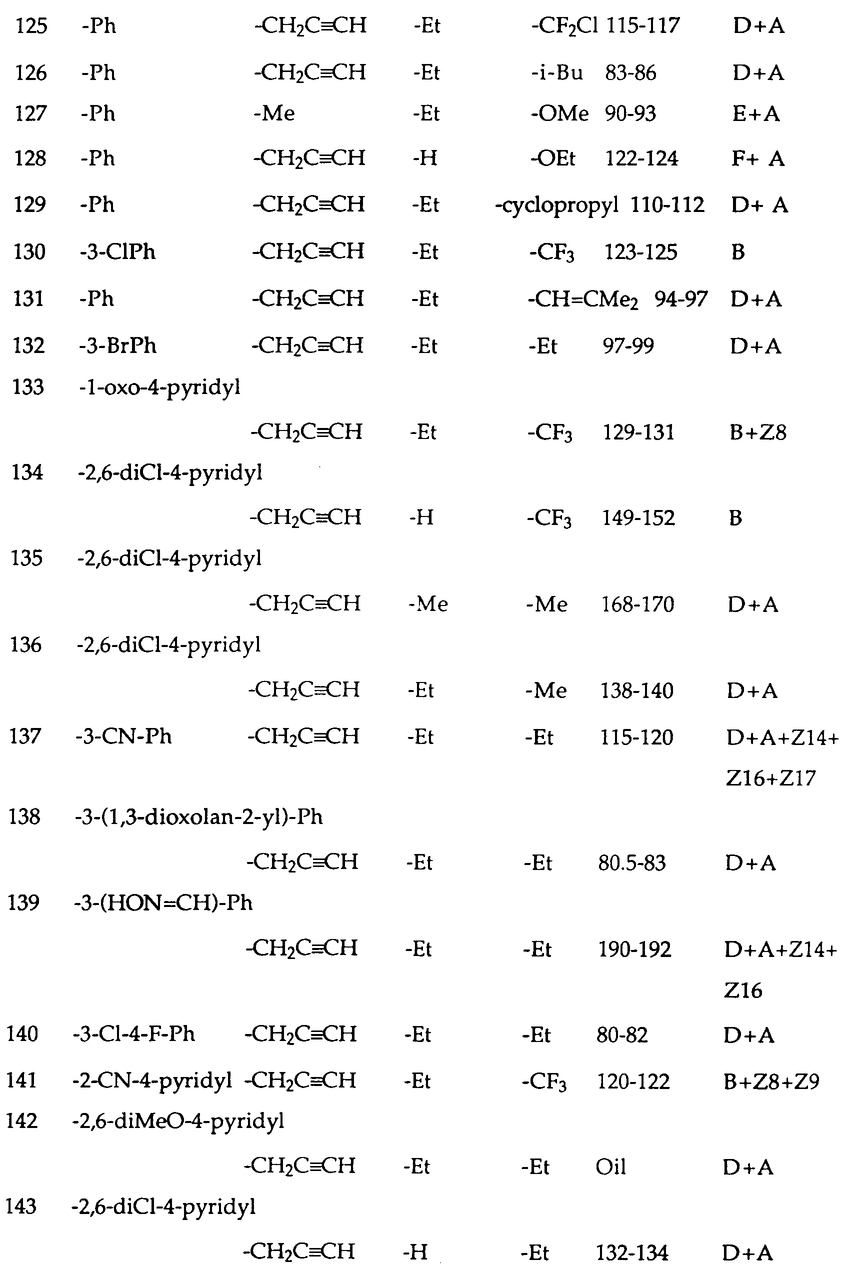

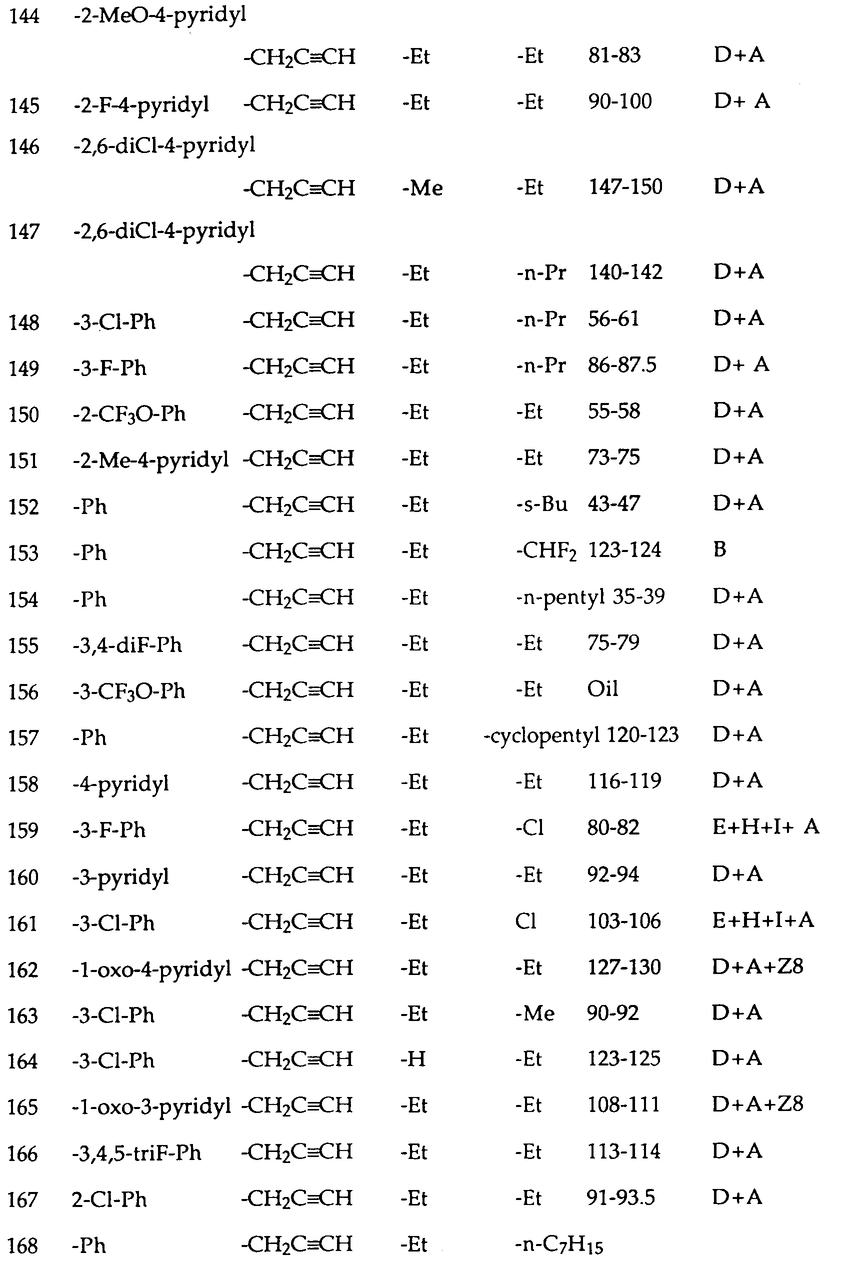

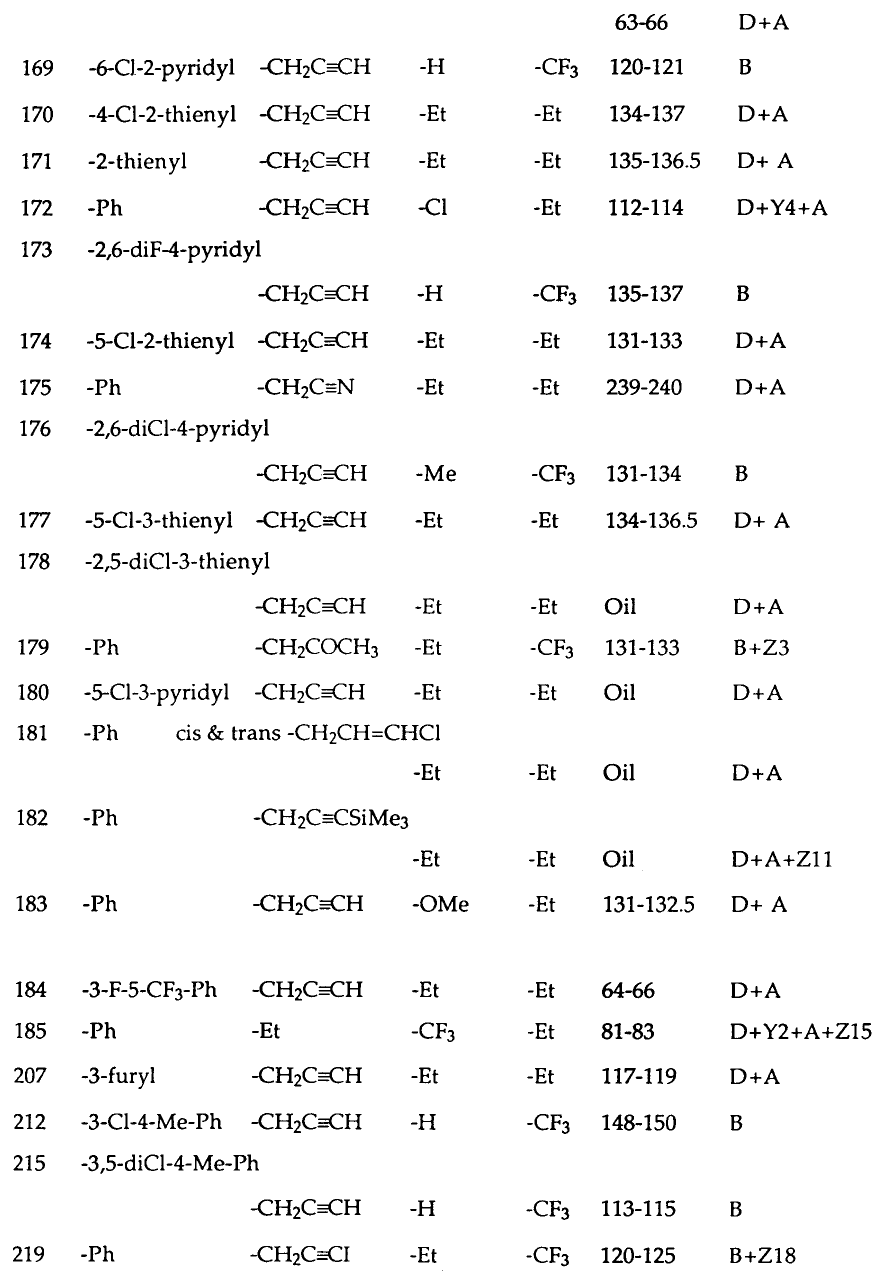

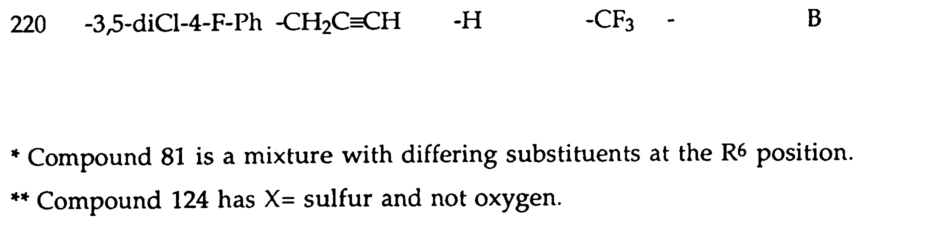

**TABLE 1A**

| Compound | R₂ | R³ | - R⁵-R⁶- link | MP °C | Synthesis |
|---|---|---|---|---|---|
| 221 | -Phenyl | -CH₂C≡CH | -CH₂CH₂CH₂- | 133-138 | B + A |
| 222 | -Phenyl | -CH₂C≡CH | -SCH=CH- * | 183-185 | C + A |
| 223 | -Phenyl | -CH₂C≡CH | -CH=C(Et)S- ** | 115-117 | C + A |
| 224 | -2-Furyl | -CH₂C≡CH | -CH₂CH₂CH₂CH₂- | 112-114 | A |
| 225 | -Phenyl | -CH₂C≡CH | -CH=CHCH=CH- | 199-201 | C + A |
| 226 | -Phenyl | -CH₂C≡CH | -CH₂CH₂CH₂CH₂- | 137-141 | B + A |

| | | | | | |
|---|---|---|---|---|---|
| * the sulfur atom (S) is bonded to the carbon atom at the 5 position of the pyrimidine ring | | | | | |
| ** the sulfur atom (S) is bonded to the carbon atom at the 6 position of the pyrimidine ring | | | | | |

The following ¹H-NMR data is provided for compounds in the above tables which were oils or solids whose melting points were not determined. These spectra were recorded at 200 MHz in CDCl₃. Chemical shifts are expressed in parts per million downfield of tetramethylsilane, which was used as standard.

| Compound No. | ¹H-NMR |
|---|---|
| 11 | 1.25(9H,s), 2.35(1H,t), 4.6(2H,d), 6.5(1H,s), 7.55(3H,m), 7.7(2H,m) |
| 18 | 4.6(2H,m), 5.0(1H,dd), 5.25(1H,dd), 5.9(1H,m), 6.9(1H,s), 7.55(5H,m) |
| 20 | 1.25(3H,t), 4.0(2H,q), 6.85(1H,s), 7.5(5H,m) |
| 62 | 1.25(3H,t), 2.5(1H,t), 2.80(2H,q), 4.65(2H,d), 7.35(1H,s), 7.6(1H,d), 8.5(1H,d) |
| 67 | 1.15(3H,t), 1.2(6H,d), 2.5(1H,t), 2.65(2H,q), 3.15(1H,m), 4.6(2H,d), 7.65(2H,s) |
| 81 | 2.4(1H,t), 4.3(2H,s), 4.6(2H,d), 6.65(1H,s), 7.55(3H,m), 7.7(2H,m) [6-CH₂Br] |
| | 2.4(1H,t), 4.4(2H,s), 4.6(2H,d), 6.71(1H,s), 7.55(3H,m), 7.7(2H,m) [6-CH₂Cl] |
| 92 | 0.25(9H,s), 1.25(3H,t), 2.37(1H,t), 2.85(2H,q), 4.60(2H,d), 7.55(3H,m), 7.75(2H,m) |
| 120 | 1.25(3H,t), 2.43(1H,t), 2.61(2H,q), 4.68(2H,d), 7.5(2H,d), 7.55(3H,m), 7.75(2H,m), 8.75(2H) |
| 124 | 1.25(9H,m), 2.7(2H,q), 3.08(2H,q), 4.62(2H,q), 7.5(5H,m) |
| 142 | 1.15(3H,t), 1.25(3H,t), 2.35(1H,t), 2.60(4H,q), 3.95(6H,s), 4.55(2H,d), 6.55(2H,s). |
| 156 | 1.25(6H,m), 2.4(1H,t), 2.65(4H,q), 4.6(2H,d), 7.35 - 7.75(4H,m) |
| 178 | 1.35(6H,m), 2.78(4H,m), 2.95(1H,t), 4.9(2H,d), 8.2(1H,s) |
| 180 | 1.20(3H,t), 1.25(3H,t), 2.45(1H,t), 2.65(4H,q), 4.60(2H,d), 8.10(1H,s), 8.75(1H,s), 8.85(1H,s). |
| 181 | 1.2(6H,m), 2.65(4H,m), 4.5(2H,m), 5.95(2H,m), 7.5(5H,m), {cis} 1.2(6 H,m), 2.65(4H,m), 4.7(2H,m), 5.95(1H,m), 6.15(1H,m), 7.5(5H,m) {trans} |
| 182 | 0.18(9H,s), 1.25(6H,m), 2,65(4H,q), 4.6(2H,s), 7.42-7.8(5H,m) |
| 220 | 2.5(1H,t), 4.6(2H,d)6.9(1H,s), 7.8(2H,d) |

The following table of compounds are additional compounds listed as examples within the embodiment of the present invention:

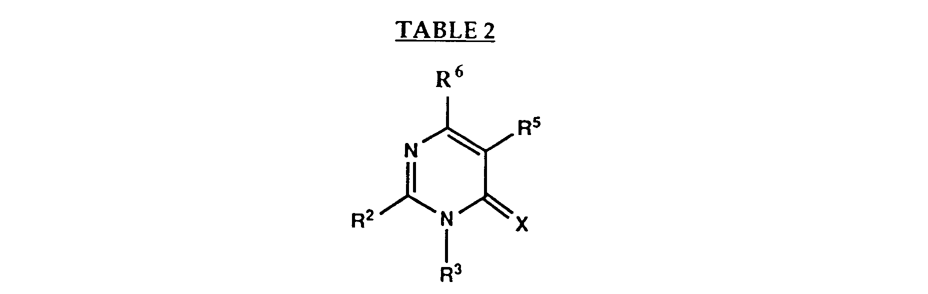

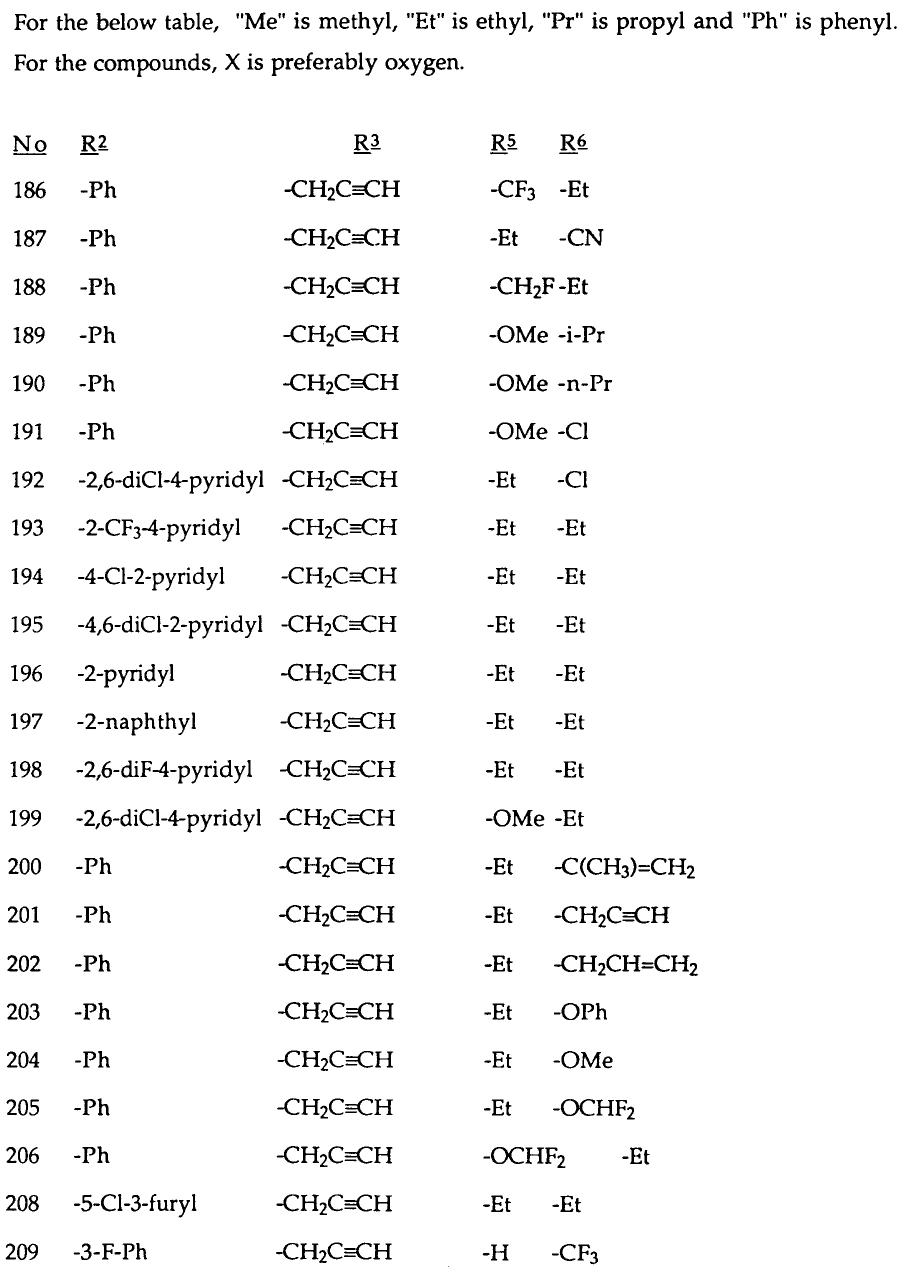

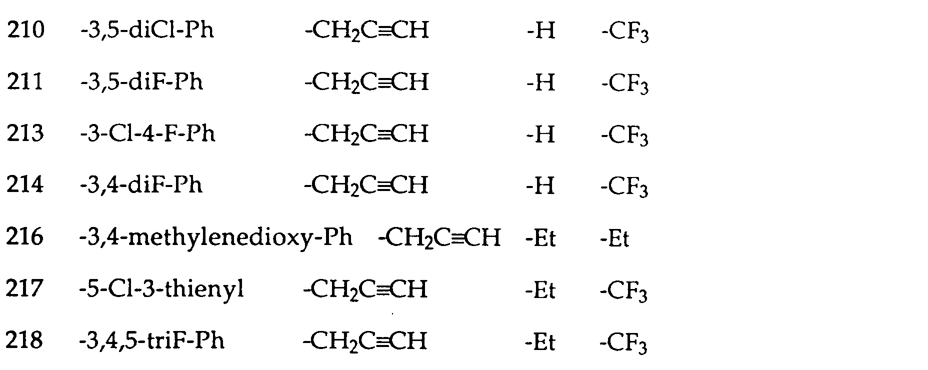

### Methods of Preparation.

The 2-arylpyrimidines of the present invention may be prepared by standard synthetic routes such as those illustrated below.

### Method A - General Description:

A precursor compound having the structure of formula I above with hydrogen (H) in the R³ substituent position is selected. Reaction with R³Y is performed in a base-solvent mixture. Y can be a halogen, alkanesulfonate, haloalkanesulfonate or optionally substituted benzenesulfonate. The bases can be sodium hydride, alkali metal hydroxides, alkali metal carbonates or alkali metal alkoxides. The solvent can be alcohol, ketone, water, ether, DMSO or DMF. A mixture of N- and O- alkylated products results.

### Method A - Specific Example 1 -Preparation of 6-ethyl-5-methyl-2-phenyl-3-propargyl-4(3H)-pyrimidinone (Compound 5):

To a stirred solution of 24.30g (0.45 mol) of sodium methoxide in 400 mL of methanol was added a slurry of 61.78g (0.29 mol) of 6-ethyl-5-methyl-2-phenyl-4(3H)-pyrimidinone in 100 mL of methanol. The mixture was heated to reflux to give a clear orange solution to which 44.89g (0.30 mol) of an 80% weight solution of propargyl bromide in toluene was added. The course of the reaction was followed by GC. Refluxing was continued for 6.5h, when an additional 20.63g (0.14mol) of an 80% weight solution of propargyl bromide in toluene was added. Refluxing was continued for an additional 4.5h. The reaction mixture was allowed to cool to room temperature and 250 mL of water and 250 mL of saturated aqueous NaHCO₃ were added. The mixture was rotovaped to remove the bulk of the methanol and extracted with three 200 mL portions of ethyl acetate. The ethyl acetate extracts were combined, filtered and extracted with three 200 mL portions of 5% aq HCl. The combined aqueous HCl extracts were basified to pH 11 with 50% aqueous NaOH and extracted with three 250mL portions of ether. The combined ether extracts were washed with 100mL of brine, dried over MgSO₄ and rotovaped to leave 30.46g of a yellowish solid. This material was triturated with 100mL portions of 10, 25 and 50% ether in hexanes and recrystallized from 25mL of toluene to furnish 15.11 g. of Compound 5 as a white solid mp 115-117°C. ¹H-NMR (d⁶-DMSO) δ 1.15(3H, t, J=7.8),
2.07(3H, s), 2.58(2H, q, J=7.8), 3.32(1H, t, J=2.4), 4.55(2H, d, J=2.4), 7.58(3H, m), 7.65(2H, m).

### Method A - Specific Example 2 - Preparation of 2-phenyl-3-propargyl-3,5,6,7-tetrahydro-4H-cyclopentapyrimidin-4-one. (Compound 221)

A slurry of 8.3 g (39.1 mmol) of 2-phenyl-3,5,6,7-tetrahydro-4H-cyclopentapyrimidin-4-one in 60 mL of tetrahydrofuran was added to 1.9 g (48 mmol) of 60% sodium hydride, with external cooling. A 6.4 g (43.0 mmol) portion of 80% by weight of propargyl bromide in toluene was added to the cooled solution. The ice bath was removed and the reaction was refluxed for 6 hours. The solvent was removed *in vacuo*, ether was added to the residue and the reaction was washed 3 times with water and once with brine. The organic layer was dried over MgSO₄ and concentrated to yield 5.0 g of crude semisolid product. The crude product was dissolved in a small amount of methylene chloride and passed through a 6 inch plug of silica gel, washing with 1 L of methylene chloride. The silica gel was washed further with 1 L of ether. The ether was removed *in vacuo* to yield 0.5 g (5.1%) of 2-phenyl-3-propargyl-3,5,6,7-tetrahydro-4H-cyclopentapyrimidin-4-one (Compound 1) as a white solid. ¹H-NMR (CDCl₃) δ 2.15 (2H,m), 2.35 (1H,t), 2.95 (4H,t), 4.65 (2H,t), 7.55 (3H,m), 7.7 (2H,m).

### Method A - Specific Example 3 - Preparation of 2-phenyl-3-propargyl-[3,2-d]-thien-4(3H)-pyrimidinone (Compound 222)

A mixture of 12.95 g (48.2 mmol) of 2-phenyl-[3,2-d]thieno-4(3H)- pyrimidinone, 2.87 g (53.1 mmol) of sodium methoxide and 100 mL of methanol was heated to reflux. To the hot stirred slurry was added 7.59 g (50.9 mmol) of an 80% by weight solution of propargyl bromide in toluene. Refluxing was continued for 3.5 days. The bulk of the methanol was rotovapped off and the residue was partitioned between 150 mL of water and 150 mL of ethyl acetate. The mixture was filtered to remove unreacted 2-phenyl-[3,2-d]-thieno-4(3H)-pyrimidinone. The organic layer of the filtrate was separated and the aqueous layer was extracted with 100 mL of ethyl acetate. The combined organic extracts were washed with brine, dried over MgSO₄ and concentrated to afford 3.03 g of a solid. This material was purified by flash chromatography on a column of 50 g of silica gel, eluting with 20, 30, 40, 50, 75 and 100% ether in hexanes to give 1.27 g of crude product. This crude product was taken up in 150 mL of ethyl acetate, washed with three 50 mL portions of 5% aqueous hydrochloric acid and 50 mL of saturated aqueous NaHCO₃, and dried over MgSO₄. Removal of the solvent left 0.89 g (7%) of 2-phenyl-3-propargyl-[3,2-d]-thieno-4(3H)-
pyrimidinone (Compound 2) as an off-white solid, mp 183 - 185^{°}C. ¹H-NMR (CDC1₃) δ 2.35(1H,t), 4.7(2H,d), 7.35(1H,d), 7.55(3H), 7.7(1H,d).

### Method B - General Description:

Direct condensation of an N-alkylamidine and a beta-keto ester is performed by warming the reagents in a solvent such as THF or neat:

R²C(=NH)N(H)R³ + R⁶C(=O)CH(R⁵)C(=O)OR --> 2-arylpyrimidine (Figure I)

Preferably R³ is a non-reactive group and when R³ is a propargyl group, preferably R⁶ is CF₃.

### Method B - Specific Example - Preparation of 5-ethyl-2-phenyl-3-propargyl-6-trifluoromethyl-4(3H)-pyrimidinone

To a stirred solution of 22.66g (0.13mol) of methyl benzimidate hydrochloride in 80mL of methanol was added 11.09g (0.13mol) of powdered sodium bicarbonate. The mixture was stirred for 0.5h and 9.1mL (0.13mol) of propargylamine was added. The mixture was stirred for 4h at room temperature and then rotovapped to remove most of the methanol. To the oily orange residue was added 34.00g of 80% pure ethyl 2-trifluoroacetylbutyrate (0.13mol). The mixture was heated at 55 - 60°C for 40h. The mixture was diluted with 300mL of ether, washed with two 150mL portions of 5% aqueous HCl and 150mL of saturated aqueous sodium bicarbonate and dried over MgSO₄. Removal of the solvent and drying at 50°C under high vacuum afforded 22.58g of an orange solid. This material was purified by flash chromatography on 325g of silica gel, eluting with 0, 10, 20, 30 and finally 40% ether in hexanes to furnish 16.86g of a yellow solid. This material was triturated with two 100mL portions of boiling hexanes and recrystallized from 150mL of 10% ether in hexanes to give 10.31g (26%) of 5-ethyl-2-phenyl-3-propargyl-6-trifluoromethyl-4(3H)-pyrimidinone (Compound No. 46) as a white solid, mp 111 - 114°C. ¹H-NMR (CDCl₃) δ 1.24(3H,t), 2.38(1H,t), 2.75(2H,q), 4.62(2H,d), 7.55(3H,m), 7.74(2H,m).

### Method C (5,6-fused ring pyrimidinones) - General Description

A 3-aroylaminoacrylamide derivative is cyclized with base to give a pyrimidinone. The starting material is prepared by acylation of the corresponding 3-aminoacrylamide or by acylation of a 3-aminoacrylate ester followed by ammonolysis:

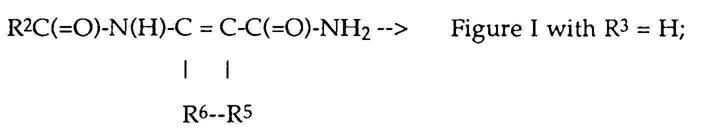

### Method C - Specific Examples - Preparation of 2-phenyl-[3,2-d]-thieno-4(3H)-pyrimidinone

(a) A stirred solution of 19.05g (0.12mol) of methyl 3-amino-2-thiophenecarboxylate and 12mL of pyridine in 200mL of dichloromethane was cooled in an ice bath and 15.5mL (0.13mol) of benzoyl chloride was added dropwise over 15min. The mixture was stirred in the ice bath for 15min and then at room temperature for 4h. The solvent was removed on the rotovap and the residue was partitioned between 100mL of 5% aqueous hydrochloric acid and three 100mL portions of ether. The combined organic extracts were washed with 50mL of 5% aqueous hydrochloric acid and 50mL of saturated aqueous sodium bicarbonate, and dried over MgSO₄. Removal of the solvent left 30.73g (98%) of crude methyl 3-(benzoylamino)-2-thiophenecarboxylate. ¹H-NMR (CDCl₃) δ 3.90(3H,s), 7.50(4H,m), 8.00(2H,m),
(b) To a stirred solution of 14.21g (54.4mmol) of crude methyl 3-(benzoylamino)-2-thiophenecarboxylate in 100mL of methanol and 100mL of THF was added 100mL of concentrated aqueous ammonia. The mixture was stirred at room temperature for 5 days and an additional 100mL of concentrated aqueous ammonia was added. After stirring for 5 more days the mixture was rotovapped to remove the bulk of the organic solvents and the residue was extracted with two 125mL portions of ethyl acetate. The insoluble material was collected by filtration and combined with the residue from evaporating the ethyl acetate extracts to dryness to furnish 11.96g (89%) of crude 3-(benzoylamino)-2-thiophenecarboxamide as a tan solid. ¹H-NMR (d6-DMSO) δ 7.55(3H,m), 7.7(1H,d), 7.95(2H,m), 8.18(1H,d).
(c) A suspension of 11.85g (48.2mmol) of crude 3-(benzoylamino)-2-thiophenecarboxamide in 100mL of 5% aqueous NaOH was refluxed for 0.5h. The mixture was allowed to cool and neutralized by addition of conc HCl. The mixture was filtered and 12.95g of wet 2-phenyl-[3,2-d]thieno-4(3H)-pyrimidinone was collected. This material was used without drying or purification. ¹H-NMR (d6-DMSO) δ 7.45(1H,d), 7.55(3H,m), 8.18(3H,m).

### Method D - General Description

An amidine hydrochloride or other salt is heated with a beta-keto ester in a solvent in the presence of a base to neutralize the hydrochloric acid. Solvents usable include xylene or toluene, preferably, or ethanol or heptane. Sodium acetate or sodium ethoxide can be the base:

R²C(=NH)NH₂ + R⁶C(=O)CH(R⁵)C(=O)OR -->Figure I with R³ = H;

precursor for Method A

### Method D - Preparation of 6-ethyl-5-methyl-2-phenyl-4(3H)-pyrimidinone.

A 2L 3-neck flask equipped with mechanical stirrer, Dean Stark trap and a reflux condenser was purged with nitrogen and charged with 93.91 g. (0.59 mol) of methyl 2-propionylpropionate, 103.58g (≦0.66mol) of benzamidine hydrochloride hydrate, 54.30g (0.66mol) of anhydrous sodium acetate and 1L of xylenes. The mixture was stirred and heated to reflux under a nitrogen atmosphere for 46 h.

The Dean Stark trap was replaced with a distillation head and about 75% of the solvent was distilled off at atmospheric pressure. After the flask had cooled to room temperature, 500mL of water and 200mL of ether were added. The mixture was filtered and the solid collected was washed thoroughly with water and ether and dried in a vacuum oven at 60°C to give 74.73g (50%) of the desired product, mp 195-199°C. ¹H-NMR (d⁶-DMSO) δ 1.23(3H, t, J=7.8), 2.02(3H, s), 2.61(2H, q, J=7.8), 7.50 (3H,m), 8.13(2H, m).

The filtrate was separated into aqueous and organic layers. The organic layer was extracted with three 125mL portions of 5% aqueous NaOH. The combined aqueous base extracts were acidified to pH 7 with aqueous HCl, allowed to cool and filtered. The solid collected was washed with water and ether and dried in a vacuum oven at 60°C to afford an additional 5.16g (4%) of the desired product, mp 196-199°C.

### Method D - preparation of 2-phenyl-3,5,6,7-tetrahydro-4H-cyclopentapyrimidin-4-one

A mixture of 15.6 g (190 mmol) of sodium acetate, 25 mL (172.7 mmol) of ethyl cyclopentanone-2-carboxylate, 27.7 g (177 mmol) of benzamidine hydrochloride and 250 mL of xylene was refluxed with a Dean Stark trap for 8 hours. Most of the xylene was distilled off and the hot reaction mixture was poured onto crushed ice. The reaction mixture was left to stand at room temperature for 0.5 hour, then vacuum filtered to remove the product, as a gray solid. The solid was air dried overnight to yield 13.35 g (37.0%) of 2-phenyl-3,5,6,7-tetrahydro-4H-cyclopentapyrimidin-4-one. ¹H-NMR (CDCl₃) δ 2.11 (2H,m), 2.90 (4H,m), 7.5 (3H,m), 8.18 (2H,m).

### Method E - General Description

Malonate diester is condensed with an amidine under basic conditions. For example, sodium methoxide in refluxing methanol may be used:

R²C(=NH)NH₂ + ROC(=O)CH(R⁵)C(=O)OR --> Figure I with R³ = H and R⁶ = OH.

### Method E - Preparation of 5-ethyl-6-hydroxy-2-phenyl-4(3H)-pyrimidinone

A mixture of 45.19g (0.29mol) of benzamidine hydrochloride hydrate, 127.42g (0.59mol) of 25% sodium methoxide in methanol, 55mL (0.29mol) of diethyl ethylmalonate and 175mL of methanol was heated at reflux for 25h. The mixture was rotovapped to remove the bulk of the methanol. The residue was diluted with 300mL of water and the pH was adjusted to 7 with concentrated hydrochloric acid. The solid precipitate was collected by filtration and dried under vacuum at 50°C to afford 31.89g (51%) of crude 5-ethyl-6-hydroxy-2-phenyl-4(3H)-pyrimidinone as a pale yellow solid. ¹H-NMR (d6-DMSO) δ 1.05(3H,t), 2.39(2H,q), 7.5(3H,m), 8.1(2H,m).

### Method F - General Description

Method F is similar to Method D except that a 3-alkoxyacrylate ester is used instead of a beta-keto ester :

R²C(=NH)]NH₂ + RO(R⁶)C=C(R⁵)C(=O)OR --> Figure I with R³ = H

Various conditions are usable : for example, amidine hydrochloride/3-alkoxyacrylate in NaOAc/DMSO at 120°C or in sodium methoxide/ethanol at 5°C.

### Method F - Specific Example - Preparation of 6-ethoxy-2-phenyl-4(3H)-pyrimidinone

A mixture of 3.14g (20.0mmol) of benzamidine hydrochloride hydrate, 1.65g (20.1mmol) of powdered anhydrous sodium acetate, 4.17g (22.2mmol) of ethyl 3,3-diethoxyacrylate and 10mL of DMSO was heated at 120°C for 8h. The mixture was cooled, diluted with 50mL of 5% aqueous NaOH and washed with two 100mL portions of ether. The aqueous layer was acidified with concentrated hydrochloric acid and the precipitate was collected by filtration and dried under vacuum at 50°C to furnish 2.28g (57%) of crude 6-ethoxy-2-phenyl-4(3H)-pyrimidinone as a yellow solid ¹H-NMR (d6-DMSO) δ 1.35(3H,t), 4.33(2H,q), 5.60(1H,s), 7.50(3H,m), 8.2(2H,m).

### Method H - General Description

6-Hydroxy-4(3H)-pyrimidinones were heated with phosphorus oxyhalide with or without a cosolvent to give 6-halo-4(3H)-pyrimidinones. For example, phosphorous oxybromide was used with an inert solvent (1,2-dichloroethane) at reflux. See U. S. Patent 4,617,393.

### Method H - Specific Example - Preparation of 4,6-dibromo-5-ethyl-2 phenylpyrimidine

A mixture of 24.50g (85.3mmol) of phosphorus oxybromide, 7.56g (37.3mmol) of 5-ethyl-6-hydroxy-2-phenyl-4(3H)-pyrimidinone and 20mL of 1,2-dichloroethane was heated at reflux for 2h. After cooling to room temperature, the mixture was poured onto 300g of crushed ice. The ice was allowed to melt, the mixture was basified by cautious addition of solid Na₂CO₃ and then extracted with two 200mL portions of ethyl acetate. The combined ethyl acetate extracts were dried over MgSO₄ and concentrated to afford 11.79g (92%) of crude 4,6-dibromo-5-ethyl- 2-phenylpyrimidine. This material was recrystallized from hexanes to furnish 6.62g (52%) of pure product, mp 101 - 103°C. ¹H-NMR (CDCl₃) δ 1.20(3H,t), 2.95(2H,q), 7.45(3H,m), 8.35(2H,m).

### Method I - General Description

4,6-dihalopyrimidines are acidically hydrolyzed to give 6-halo-4(3H)-pyrimidinones. See U. S. Patent 4,617,393.

### Method I - Specific Example - Preparation of 6-bromo-5-ethyl-2-phenyl-4(3H)-pyrimidinone

To 8.29g (26.1mmol) of crude 4,6-dibromo-5-ethyl-2-phenylpyrimidine was added a mixture of 4mL of water and 15mL of concentrated sulfuric acid. The mixture was stirred for 18h and poured onto 200g of crushed ice. After the ice had melted the precipitate was collected by filtration and dried under vacuum to afford 7.21g (99%) of crude 6-bromo-5-ethyl-2-phenyl-4(3H)-pyrimidinone. ¹H-NMR (d6-DMSO) δ 1.10(3H,t), 2.55(2H,q), 7.55(3H,m), 8.10(2H,m).

### Method Y1

### (a) Preparation of 5-bromo-2-phenyl-6-trifluoromethyl-4(3H)-pyrimidinone.

To a solution of 1.0 g (3.94 mmol) of 2-phenyl-6-trifluoromethyl-4(3H)-pyrimidinone and 20 mL of glacial acetic acid was added 1.0 g (5.6 mmol) N-bromosuccinimide and the mixture was left to stir at room temperature for 16h. The reaction was poured onto ice water and vacuum filtered, washing well with water. The crude product was recrystallized from ethyl acetate to yield 1.05g (83.5%) of 5-bromo-2-phenyl-6-trifluoromethyl-4(3H)-pyrimidinone, as a white solid. ¹H-NMR (d₆DMSO) δ 7.6 (3H, m); 8.15 (2H, m).

### (b) Preparation of 5-bromo-2,6-diphenyl-4(3H)-pyrimidinone.

To a suspension of 13.37 g (56 mmol) of 2,6-diphenyl-4(3H)-pyrimidinone and 200 mL glacial acetic acid was added 15.1 g (84.8 mmol) of N-bromosuccinimide and the mixture was left to stir at room temperature for 60h. The reaction was poured onto 100 g crushed ice and vacuum filtered, washing well with water, then air dried to yield 8.85 g (48%) 5-bromo-2,6-diphenyl-4(3H)-pyrimidinone, as a white solid. ¹H-NMR (d₆DMSO) δ 7.55 (6H, m); 7.75 (2H, m); 8.15 (2H, m).

### Method Y2 - Preparation of 6-ethyl-5-iodo-2-phenyl-4(3H)-pyrimidinone

A mixture of 8.18g (40.9mmol) of 6-ethyl-2-phenyl-4(3H)-pyrimidinone, 1.68g (42.0mmol) of sodium hydroxide, 10.42g (41.0mmol) of iodine and 50mL of water was heated at 50°C for 4h. The mixture was cooled and filtered. The white solid collected was dried in a vacuum oven to leave 12.60g (75%) of 6-ethyl-5-iodo-2-phenyl-4(3H)-pyrimidinone. ¹H-NMR (d6-DMSO) δ 1.25(3H,t), 2.85(2H,q), 7.50(3H,m), 8.15(2H,m).

### Method Y3 - Preparation of 4,6-difluoro-5-ethyl-2-phenyl-pyrimidinone.

To a stirred solution of 3.14g (12.41mmol) portion of 4,6-dichloro-5-ethyl-2-phenylpyrimidine in 25mL of sulfolane at 70 - 80°C was added 6.37g (109.8mmol) of spray-dried potassium fluoride. The mixture was heated at 200°C for 0.5h. After cooling, the mixture was diluted with 100mL of water and extracted with 400mL of 1:1 ether:hexanes. The organic layer was washed with two 100mL portions of water, dried over MgSO₄ and concentrated to give 2.30g of crude product. This material was combined with 0.29g of crude product from another run and purified by flash chromatography on a column of 40g of silica gel. The column was eluted with 0, 5, 10, 15 and 20% ether in hexanes to furnish 2.18g (71%) of 4,6-difluoro-5-ethyl-2-phenylpyrimidine as a white solid, m.p. 49 - 51°C. ¹H-NMR (CDCl₃) δ 1.2(3H,t), 2.65(2H,q), 7.50(3H,m), 8.4(2H,m).

### Method Y4 - Preparation of 5-Chloro-6-ethyl-2-phenyl-4(3H)-pyrimidinone

A stirred solution of 7.81g (39.1mmol) of 6-ethyl-2-phenyl-4(3H)-pyrimidinone and 5.80g (43.4mmol) of N-chlorosuccinimide in 100mL of glacial acetic acid was heated at 90°C for 4h. The mixture was cooled, poured onto crushed ice and allowed to stand until the ice had melted. The mixture was filtered and the solid collected was washed with water and a little ether. The solid was dried in a vacuum oven at 50C to afford 7.99g of 5-chloro-6-ethyl-2-phenyl-4(3H)-pyrimidinone (an intermediate for compound 172) as a white solid. ¹H-NMR (d6-DMSO) 1.30(3H,t), 2.8(2H,q), 7.6(3H,m), 8.2(2H.m).

### Method Z1 - Preparation of 6-dimethylaminocarbonyl-5-methyl-2-phenyl-3-propargyl-4(3H)-pyrimidinone. (Compound 19)

To a solution of 1.81g (6.1mmol) of 6-ethoxycarbonyl-5-methyl-2-phenyl-3-propargyl-4(3H)-pyrimidinone in 100mL of ethanol and 50mL of THF was added 50mL of 5% aqueous sodium hydroxide. The mixture was stirred at room temperature for 24h and rotovapped to remove the bulk of the organic solvents. The residue was diluted with 50mL of 5% aqueous sodium hydroxide and washed with 100mL of ether. The aqueous phase was acidified with concentrated hydrochloric acid and extracted with two 100mL portions of ethyl acetate. The combined ethyl acetate extracts were washed with 50mL of brine, dried over MgSO₄ and concentrated to leave 0.87g (53%) of crude 6-carboxy-5-methyl-2-phenyl-3-propargyl-4(3H)-pyrimidinone as a brown oil.

To a stirred solution of 0.87g (3.2mmol) of crude 6-carboxy-5-methyl-2-phenyl-3-propargyl-4(3H)-pyrimidinone, 0.32g (3.9mmol) of dimethylamine hydrochloride and 2mL of pyridine in 10mL of THF was added 0.74g (3.6mmol) of solid N,N'-dicyclohexylcarbodiimide. The mixture was stirred at room temperature for 4 days and filtered to remove insoluble material. The filtrate was diluted with 150mL of ethyl acetate, washed with 50mL of 5% aqueous HCl and 50mL of saturated aqueous sodium bicarbonate and dried over MgSO₄. Removal of the solvent left 0.40g of crude product which was purified by flash chromatography on a 30g column of silica gel, eluted with 60, 80 and 100% ethyl acetate in hexanes to furnish 0.30g (32%) of 6-dimethylaminocarbonyl-5-methyl-2-phenyl-3-propargyl-4(3H)-pyrimidinone (compound 19), mp 137 - 140°C. ¹H-NMR (CDCl₃) δ 2.15(3H,s), 2.40(1H,t), 3.00(3H,s), 3.10(3H,s), 4.65(2H,d), 7.55(3H,m), 7.70 (2H,m).

### Method Z2 - Preparation of 6-dimethylamino-5-methyl-2-phenyl-3-propargyl-4(3H)-pyrimidinone. (Compound 23)

To an ice cooled solution of 1.5 g (3.8 mmol) 6-chloro-5-methyl-2-phenyl-3-propargyl-4(3H)-pyrimidinone in 4 mL of tetrahydrofuran, was added 22 mL (99 mmol) of 4.5 M dimethylamine in ether portionwise (2-4 mL) over a period of 7 days. The reaction mixture was allowed to warm and stir at room temperature after each addition. The progress of the reaction was followed by gas chromatography and proceeded to 80% completion. The solvent was removed *in vacuo* and the residue was taken up in ether and washed twice with water. The organic layer was dried over MgSO₄ and concentrated to yield 1.15 g crude solid product. Flash column chromatography on silica gel (gradient elution 25-30% ethyl acetatehexane) afforded pure 6-dimethylamino-5-methyl-2-phenyl-3-propargyl-4(3H)-pyrimidinone (compound 23), as a white solid. ¹H-NMR (CDCl₃) δ 2.2 (3H, s); 2.35 (1H, t); 3.5 (6H, s); 4.6 (2H, d); 7.65 (3H, m); 7.75 (2H, m).

### Method Z3 - Preparation of 5-Ethyl-3-(2-oxopropyl)-2-phenyl-6-trifluoromethyl-4(3H)-pyrimidinone (Compound 179)

To a stirred solution of 4.83g (15.8mmol) of 5-ethyl-3-propargyl-2-phenyl-6-trifluoromethyl-4(3H)-pyrimidinone (compound 46) in 50mL of THF was added 50mL of 10% aq NaOH. The mixture was heated at reflux for 2h, cooled and diluted with 150mL of ethyl acetate. The organic layer was separated, washed with 50mL of water and 50mL of brine and dried over MgSO₄. Removal of the solvent afforded 4.74g of 5-ethyl-3-(2-oxopropyl)-2-phenyl-6-trifluoromethyl-4(3H)-pyrimidinone (compound 179) as a white solid. ¹H-NMR (CDCl₃) 1.2(3H), 2.2(3H,s), 2.7(2H,q), 4.7(2H,s), 7.45(5H,m).

### Method Z4 - Preparation of 5-methyl-2-phenyl-3-propargyl-6-methylthio-4(3H)-pyrimidinone. (Compound 26)

To a solution of 2.5 g (9.67 mmol) of 6-chloro-5-methyl-2-phenyl-3-propargyl-4(3H)-pyrimidinone in 100 mL of methanol, was added 0.8 g (11.4 mmol) sodium thiomethoxide and the reaction was stirred at room temperature for 4 days. The methanol was evaporated and the residue was dissolved in 100 mL of ethyl acetate, then washed three times with 50 mL of 1 M sodium hydroxide followed by one time with 50 mL of brine. The organic layer was dried over MgSO₄ and concentrated to yield 2.6 g crude product. Flash column chromatography on silica gel (100% methylene chloride) afforded 5-methyl-2-phenyl-3-propargyl-6-thiomethyl-4(3H)-pyrimidinone (Compound 26) as a white solid. ¹H-NMR (CDCl₃) δ 2.1 (3H, s); 2.35 (1H, t); 2.5 (3H, s); 4.55 (2H, d); 7.5 (3H, m); 7.7 (2H, m).

### Method Z5 - Preparation of 3-(2,2-dimethoxypropyl)-6-ethyl-5-methyl-2-phenyl-4(3H)-pyrimidinone (Compound 36)

To a stirred suspension of 4.51g (17.9mmol) of 6-ethyl-5-methyl-2-phenyl-3-propargyl-4(3H)-pyrimidinone (Compound 5) in 30mL of methanol was added 7.50g (34.7mmol) of a 25% by weight solution of sodium methoxide in methanol. The mixture was warmed until homogeneous and 2.2mL (35.3mmol) of methyl iodide was added. The mixture was refluxed for 4h and then rotovapped to remove the bulk of the methanol. The residue was partitioned between 100mL of water and two 100mL portions of ether. The combined ether layers were washed with 50mL of brine and dried over MgSO₄. Removal of the solvent afforded 4.35g of a yellow oil. Flash chromatography on a column of 50g of silica gel, eluting with 20, 30, 40 and 50% ether in hexanes furnished 3.30g (58%) of 3-(2,2-dimethoxypropyl)-6-ethyl-5-methyl-2-phenyl-4(3H)-pyrimidinone (compound 36), mp 80 - 83°C. ¹H-NMR (CDCl₃) δ 1.15(3H,s), 1.25(3H,t), 2.15(3H,s), 2.65(2H,q), 2.85(6H,s), 4.4(2H,m), 7.45(5H,s).

### Method Z6 - Preparation of 3-methoxymethyl-2-phenyl-6-trifluoromethyl-4(3H)-pyrimidinone (Compound 6)

To a solution of 1.5 g (5.9 mmol) of 2-phenyl-6-trifluoromethyl-4(3H)-pyrimidinone, 17.2 g (226.3 mmol) dimethoxymethane, and 35 mL of chloroform was added 2.5 g (17.6 mmol) phosphorous pentoxide, at room temperature. By TLC (25% ethyl acetate in hexane) the reaction was incomplete after 4h and an additional 3 g (21.1 mmol) phosphorous pentoxide was added. Stirring was continued for 16h. The reaction mixture was poured onto crushed ice and 1 M sodium hydroxide and methylene chloride were added. The layers were separated and the aqueous layer was extracted twice with methylene chloride. The organic extracts were combined and washed with brine, then dried over MgSO₄ and concentrated to yield 1.1 g crude product, which was purified by recrystallization from hexane. Thus, 0.55 g (32%) 3-methoxymethyl-2-phenyl-6-trifiuoromethyl-4(3H)-pyrimidinone (Compound 6) as a yellow solid was obtained. ¹H-NMR (CDCl₃) δ 3.55 (3H, s); 5.2 (2H, d); 6.85 (1H, s); 7.65 (3H, m); 7.75 (2H, m).

### Method Z7 - Preparation of 6-ethyl-2-phenyl-3-propargyl-5-(2-trimethylsilylethynyl)-4(3H)-pyrimidinone (Compound 92)

To a stirred solution of 3.59g (9.86mmol) of 6-ethyl-5-iodo-2-phenyl-3-propargyl-4(3H)-pyrimidinone and 16.45g (167.5mmol) of trimethylsilylacetylene in 40mL of DMF were added 1.13g (0.98mmol) of tetrakis (triphenylphosphine)palladium(0), 0.41g (2.15mmol) of copper (I) iodide and 2.8mL (20.0mmol) of triethylamine. The mixture was stirred at room temperature for 18h, diluted with 200mL of water and extracted with two 200mL portions of ether. The combined ether extracts were dried over MgSO₄ and evaporated under reduced pressure to leave 5.71g of a black oil. This material was subjected to flash chromatography on a column of 50g of silica gel, eluted with 0, 10, 20, 30, 40, 50, 60 and 80% ether in hexanes to afford 0.80g of material. Further purification was effected by chromatography on a column of activity I alumina eluted with 0, 10, 20, 35, 50, 75 and 100% ether in hexanes. This process yielded 0.43g (13%) of 6-ethyl-2-phenyl-3-propargyl-5-(2-trimethylsilylethynyl)-4(3H)-pyrimidinone as an oil. ¹H-NMR (CDCl3) δ 0.25(9H,s), 1.25(3H,t), 2.37(1H,t), 2.85(2H,q), 4.60(2H,d), 7.55(3H,m), 7.75(2H,m).

### Method Z8 - 5-Ethyl-2-(1-oxo-4-pyridyl)-3-propargyl-6-trifluoromethyl-4(3H)-pyrimidinone (Compound 133)

To a stirred suspension of 8.54g (27.8mmol) of -5-ethyl-3-propargyl-2-(4-pyridyl)-6-trifluoromethyl-4(3H)-pyrimidinone (compound 58) in 50mL of ethanol was adsded 9.07g (18.3mmol) of monoperoxyphthalic acid magnesium salt hexahydrate. The mixture was stirred at room temperature for 24h. The bulk of the ethanol was removed on the rotovap and the residue was partitioned between 150mL of ethyl acetate and 75mL of 5% aqueous hydrochloric acid. The organic layer was washed with two 75mL portions of saturated aqueous NaHCO₃, dried MgSO₄ and concentrated to leave 8.42g of 5-ethyl-2-(1-oxo-4-pyridyl)-3-propargyl-6-trifluoromethyl-4(3H)-pyrimidinone (compound 133) as a yellow solid. ¹H-NMR (CDCl₃) 1.25(3H,t), 1.30(3H,t), 2.60(1H,t), 2.8(4H,m), 4.7(2H,d), 7.8(2H,d), 8.35(2H,d).

### Method Z9 - Preparation of 2-(2-Cyano-4-pyridyl)-5-ethyl-3-propargyl-6-trifluoromethyl-4(3H)-pyrimidinone (Compound 141)

To a stirred solution of 6.96g (21.6mmol) of 5-ethyl-2-(1-oxo-4-pyridyl)-3-propargyl-6-trifluoromethyl-4(3H)-pyrimidinone and 6.0mL (42.8mmol) of triethylamine in 20mL of acetonitrile was added 11.5mL (86.3mmol) of trimethylsilyl cyanide. The mixture was heated at reflux for 4h. After standing overnight, the mixture was diluted with 150mL of ether, washed with three 50mL portions of water and dried over MgSO₄. Removal of the solvent left 4.94g of crude product as a black tar. This material was purified by flash chromatography on 60g of silica gel, eluting with 0, 20, 35, 50, 65, 80 and 100% ether in hexanes to furnish 1.78g of 2-(2-cyan-4-pyridyl)-5-ethyl-3-propargyl-6-trifluoromethyl-4(3H)-pyrimidinone (compound 141) as a solid. ¹H-NMR (CDCl₃) 1.25(3H,t), 2.60(1H,t), 2.8(2H,q), 4.6(2H,d), 8.0(1H,d), 8.10(1H,s), 9.0(1H,d).

### Method Z10 - Preparation of 2-phenyl-3,5,6-triethyl-4(3H)-pyrimidinethione (Compound 124)

A mixture of 1.0g (3.9 mmol) 2-phenyl-3,5,6-triethyl-4(3H)-pyrimidinone, 0.87g (2.1 mmol) Lawesson's reagent and 35 mL toluene was refluxed for 20h. By TLC (20% ethyl acetate-hexane) the product was more polar than the starting material. The reaction was incomplete and an additional 1.2 g (2.96 mmol) of Lawesson's reagent was added and refluxing was continued for 16h. The solvent was removed *in vacuo* to leave 2.2 g yellow wet solid. Flash column chromatography on silica gel (20% ethyl acetate in hexane) afforded 0.5 g of material containing the thione, which was again purified by flash chromatography (5% ethyl acetate-hexane) to yield 280 mg (26.4%) of 2-phenyl-3,5,6-triethyl-4(3H)-pyrimidinthione (compound 124), as an oil. ¹H-NMR (CDCl₃) δ 1.25 (9H, m); 2.7 (2H, q); 3.05 (2H, q); 4.6 (2H, q); 7.5 (5H, m).

### Method Z11 - Preparation of 5,6-Diethyl-2-phenyl-3-(3-trimethylsilylprop2-ynyl)-4(3H)-pyrimidinone (Compound 182)

To an oven-dried 50mL 3-neck flask were charged 0.9g (3.38mmol) of 5,6-diethyl-2-phenyl-3-propargyl-4(3H)-pyrimidinone and 25mL freshly distilled THF. The solution was cooled to -70°C and 2.2mL of 1.6 M (3.52mmol) *n*-butyllithium in hexane was added at a rate to maintain the temperature below -62C during the addition. The reaction mixture turned black and was allowed to stir for 12 minutes at -70°C. A 0.47mL (3.70mmol) portion of trimethylsilyl chloride was added and the reaction stirred for 20 minutes at -70°C. The dry ice bath was removed and the reaction was left to stir and warm to room temperature overnight. The THF was removed *in vacuo* and ether was added. The ether solution was washed 3 times with water then dried over MgSO₄ and concentrated to yield 1.2g of crude product, as a brown oil. The crude product was purified by chromatography on a 30g silica gel column eluting with 18% ethyl acetate in hexane. 0.8g (70% yield) of 5,6-diethyl-2-phenyl-3-(3-trimethylsilyl-2-propynyl)-4(3H)-pyrimidinone (compound 182), was obtained as a yellow oil. ¹H-NMR (CDCl₃) 0.18(9H,s), 1.25(6H,m), 2,65(4H,q), 4.6(2H,s), 7.6(3H,m), 7.42-7.8(5H,m)

### Method Z12 - Preparation of 5-ethyl-3-(pent-2-yn-4-en-1-yl)-2-phenyl-6-trifluoromethyl-4(3H)-pyrimidinone (Compound 111)

To a deoxygenated solution of 1.01g (3.28mmol) of 5-ethyl-2-phenyl-3-propargyl-6-trifluoromethyl-4(3H)-pyrimidinone and 0.61g (3.96mmol) of vinyl iodide in 25mL of triethylamine was added a mixture of 60mg of copper (I) iodide and 60mg of bis(triphenylphosphine) palladium (II) chloride. The mixture was stirred at room temperature for 22h and rotovapped to remove the bulk of the triethylamine. The residue was taken up in 150mL of ethyl acetate, washed with 75mL of 5% aqueous hydrochloric acid, 75mL of saturated aqueous sodium bicarbonate and 75mL of brine, and dried. Removal of the solvent left 1.51g of a brown tar. Flash chromatography on a column of 30g of silica gel eluting with 20, 40, 60 and 60% ether in hexanes afforded 0.31g of crude 5-ethyl-3-(pent-2-yn-4-en-1-yl)-2-phenyl-6-trifluoromethyl-4(3H)-pyrimidinone. A second chromatography yielded 0.25g (23%) of pure 5-ethyl-3-(pent-2-yn-4-en-1-yl)-2-phenyl-6-trifluoromethyl-4(3H)-pyrimidinone (compound 111) as a solid, mp 104 - 106°C. ¹H-NMR (CDCl₃) d 1.25(3H,t), 2.8(2H,q), 4.75(2H,s), 5.5-5.9(3H,m), 7.55(3H,m), 7.7(2H,m).

### Z13 - Preparation of 2-(1-methyl-3-pyridinium)-5-ethyl-3-propargyl-6-trifluoromethyl-4(3H)-pyrimidinone iodide (Compound 61)

A solution of 1.23g (4.01mmol) of 5-ethyl-3-propargyl-2-(3-pyridyl)-6-trifluoromethyl-4(3H)-pyrimidinone and 1.0mL (16.1mmol) of methyl iodide in 5mL of CHCl₃ was heated at reflux for 6h. An additional 1.0mL portion of methyl iodide was added and refluxing was continued overnight. The mixture was rotovapped to leave 1.86g of 2-(1-methyl-3-pyridinium)-5-ethyl-3-propargyl-6-trifluoromethyl-4(3H)-pyrimidinone iodide as a brown solid. ¹H-NMR (CDCl₃) δ 1.2(3H,t), 2.7(1H,t), 2.75(2H,q), 4.65(3H,s), 4.9(2H,d), 8.4(1H,t), 8.9(1H,d), 9.2(1H,s), 9.5(1H,d).

### Method Z14- Preparation of 5,6-diethyl-2-(3-formylphenyl)-3-propargyl-4(3H)-pyrimidinone (Compound 69)

To a solution of 2.3g (6.8mmol) of 5,6-diethyl-2-[3-(2-dioxolanyl)phenyl]-3-propargyl-4(3H)-pyrimidinone in 1mL of ethyl acetate was added 50mL of 6M hydrochloric acid and the mixture was stirred for 4 hours. The reaction was followed by gas chromatography and TLC(20% ethyl acetate in hexane). Upon completion of reaction, 75mL of ether and 150 mL of water were added to the reaction mixture. The layers were separated and the aqueous layer was extracted twice with 50 mL of ether. The organic layers were combined, dried over MgSO₄ and concentrated to yield 1.73g of 5,6-diethyl-2-(3-formylphenyl)-3-propargyl-4(3H)-pyrimidinone (Compound 69) as a yellow oil (86%), which solidified on standing. Mp.= 82-86°C. ¹H-NMR (CDCl₃) δ 1.25(6H,m), 2.4(1H,t), 2.65(4H,q), 4.6(2H,d), 7.7(1H,t), 8.05(2H,m), 8.25(1H,s), 10.15(1H,s).

### Method Z15 - Preparation of 3,6-diethyl-2-phenyl-5-trifluoromethyl-4(3H)-pyrimidinone (Compound 185)

A mixture of 1.00g (2.8mmol) of 3,6-diethyl-5-iodo-2-phenyl-4(3H)-pyrimidinone, 1.08g (5.7mmol) of copper (I) iodide, 1.54g (11.3mmol) of sodium trifluoroacetate and 8mL of anhydrous N-methylpyrrolidinone was heated at 175C for 2h. The mixture was cooled, diluted with 175 mL of ether, washed with four 50mL portions of water and dried over MgSO₄. Removal of the solvent on the rotovap afforded 0.92g of crude product as a brown oil. This material was purified by flash chromatography on a 25g column of silica gel eluting with 100mL portions of 0, 10, 20, 30, 40, 50 and 75% ether in hexanes to afford 0.35g of 3,6-diethyl-2-phenyl-5-trifluoromethyl-4(3H)-pyrimidinone (compound 185) as a white solid. ¹H-NMR (CDCl₃) 1.25(3H,t), 1.30(3H,t), 2.8(2H,q), 4.0(2H,q), 7.5(5H).

### Method Z16 - Preparation of 5,6-Diethyl-2-(3-hydroxyiminophenyl)-3-propargyl-4(3H)-pyrimidinone (Compound 139)

To a 100mL RBF were charged 1.1g (3.7mmol) of 5,6-diethyl-2-(3-formyl-phenyl)-3-propargyl-4(3H)-pyrimidinone, 0.52g (7.5mmol) of hydroxylamine hydrochloride and 50mL of ethanol. The reaction mixture was refluxed for 17 hours. The ethanol was removed *in vacuo* and ether and ethyl acetate were added to the residue. The organics were washed 3 times with water. The organic layer was gravity filtered to remove 0.22g of 5,6-diethyl-2-(3-hydroxyiminophenyl)-3-propargyl-4(3H)-pyrimidinone (compound 139). The organic layer was dried over MgSO₄ and concentrated to yield a further 0.67g of 5,6-diethyl-2-(3-hydroxyiminophenyl)-3-propargyl-4(3H)-pyrimidinone (compound 139) as a white solid. A combined yield of 77.6% was obtained. ¹H-NMR (CDCl₃) 1.25(6H,m); 2.35(1H,t); 2.65(4H,m); 4.6(2H,d); 7.49-8.15(4H,m); 8.7(1H,s)

### Method Z17 - Preparation of 5,6-Diethyl-2-(3-cyanophenyl)-3-propargyl-4(3H)-pyrimidinone (Compound 137)

To an ice cooled solution of 0.64g (2.07mmol) of 5,6-diethyl-2-(3-hydroxyiminophenyl)-3-propargyl-4(3H)-pyrimidinone in 10mL methylene chloride, 1.5mL (20.5mmol) of thionyl chloride was added dropwise. The ice bath was removed and the reaction continued to stir at room temperature for 16h. The reaction mixture was concentrated and 10mL portions of methylene chloride were added and removed *in vacuo* twice. 0.65g of a light brown solid was obtained as crude product. This was combined with 0.15g crude product from a previous run. The crude product was purified by passing it through a 4 inch plug of basic alumina and washing with 700mL of methylene chloride. 400mg of 5,6-diethyl-2-(3-cyanophenyl)-3-propargyl-4(3H)-pyrimidinone (compound 137) was obtained. ¹H-NMR (CDCl₃) 1.25(6H,m); 2.4(1H,t); 2.65(4H,m); 4.58(2H,d); 7.64-8.1(4H,m).

### Method Z18 - Preparation of 5-ethyl-3-(3-iodopropargyl)-2-phenyl-6-trifluoromethyl-4(3H)-pyrimidinone (Compound 219)

A stirred solution of 1.53g (5.0mmol) of 5-ethyl-2-phenyl-3-propargyl-6-trifluoromethyl- 4(3H)-pyrimidinone (Compound 46) in 30mL of THF was cooled to -70^{°}C and 4.5mL of 1.6M n-BuLi in hexanes (7.2mmol) was added dropwise over 15min. The mixture was stirred at -70^{°}C for 45min and a solution of 1.50g (6.7mmol) of N-iodosuccinimide in 10mL of THF was added dropwise over 15min. The mixture was stirred at -70°C for 45min and at room temperature for 30min. The mixture was diluted with 175mL of ether, washed with two 50mL portions of water and 50mL of saturated aqueous NaHCO₃ and dried over MgSO₄. Removal of the solvent left 2.36g of crude product as a brown solid which was purified by flash chromatography on 30g of silica gel, eluting with 0, 10, 20, 30 and 40% ether in hexanes, to furnish 0.96g (44%) of 5-ethyl-3-(3-iodopropargyl)-2-phenyl-6-trifluoromethyl-4(3H)-pyrimidinone (Compound 219) as a an off-white solid, m.p. 120 - 125°C (dec). ¹H-NMR (CDCl₃) δ 1.25(3H,t), 2.75(2H,q), 4.75 (2H,s), 7.5 - 7.7 (5H).

### Methods of Use.

In another aspect, this invention relates to a method of controlling weeds comprising applying to said weed or the locus of said weed or to the surface of the growth medium of said weed a herbicidally effective amount of a compound of the invention.

The compounds of the invention are useful as preemergence and postemergence herbicides. In general, they require lower doses to control weeds preemergence. Preemergence herbicides are usually applied to the soil either before, during or after seeding, but before the crop emerges. Postemergence herbicides are applied after the plants have emerged and during their growth period. The embodied materials generally show selectivity to several agronomically important crops such as corn, cotton, rice, soybean, sugarbeet, sunflower, peanut and wheat.

Under some conditions the compounds of the invention may be incorporated into the soil or other growth medium prior to planting a crop. This incorporation may be by any convenient means, including mixing with the soil, applying the compound to the surface of the soil and then dishing or dragging into the soil to the desired depth, or by employing a liquid carrier.

The 2-arylpyrimidines of the present invention can be applied to various loci such the soil or the foliage. For such purposes these compounds can be used in the technical or pure form as prepared, as solutions or as formulations. The compounds are usually taken up in a carrier or are formulated so as to render them suitable for subsequent dissemination as herbicides. For example, these chemical agents can be formulated as wettable powders, emulsifiable concentrates, dusts, granular formulations, aerosols, or flowable emulsion concentrates. In such formulations, the compounds are extended with a liquid or solid carrier and, when desired, suitable surfactants are incorporated.

It is usually desirable, particularly in the case of foliar spray formulations, to include adjuvants, such as wetting agents, spreading agents, dispersing agents, stickers, adhesive and the like in accordance with agricultural practices. Such adjuvants commonly used in the art can be found in the John W. McCutcheon, Inc. publication "Detergents and Emulsifiers, Annual", Allured Publishing Company, Ridgewood, New Jersey, U.S.A.

The 2-arylpyrimidines can be applied as herbicidal sprays by methods commonly employed, such as conventional high-gallonage hydraulic sprays, low-gallonage sprays, air-blast spray, aerial sprays and dusts. The dilution and rate of application will depend upon the type of equipment employed, the method of application and weeds to be controlled, but the preferred effective amount is usually from about 0.01 lb. to about 10 lbs. per acre of the active ingredient.

As a soil treatment the chemical can be incorporated in the soil or applied to the surface usually at a rate of from about 0.01 to about 10 lbs. per acre. As a foliar spray, the toxicant is usually applied to growing plants at a rate of from about 0.01 to about 10 lbs. per acre.

The 2-arylpyrimidines of the invention can also be mixed with fertilizers or fertilizing materials before their application. In one type of solid fertilizing composition in which the arylpyrimidines can be used, particles of a fertilizer or fertilizing ingredients, such as ammonium sulfate, ammonium nitrate, or ammonium phosphate, can be coated with one or more of the compounds. The solid compounds and solid fertilizing material can also be admixed in mixing or blending equipment, or they can be incorporated with fertilizers in granular formulations. Any relative proportion of fertilizer can be used which is suitable for the crops and weeds to be treated. The 2-arylpyrimidine will commonly be from about 5% to about 25% of the fertilizing composition. These compositions provide fertilizing materials which promote the rapid growth of desired plants, and at the same time control the growth of undesired plants.

For some applications, one or more other herbicides may be added of the herbicides of the present invention, thereby providing additional advantages and effectiveness. When mixtures of herbicides are employed, the relative proportions which are used will depend upon the relative efficacy of compounds in the mixture with respect to the plants to be treated. Examples of other herbicides which can be combined with those of the present invention include:

### CARBOXYLIC ACIDS AND DERIVATIVES

2,3,6-trichlorobenzoic acid and its salts;
2,3,5,6-tetrachlorobenzoic acid and its salts;
2-methoxy-3,5,6-trichlorobenzoic acid and its salts;
2-methoxy-3,6-dichlorobenzoic acid and its salts;
2-methyl-3,6-dichlorobenzoic acid and its salts;
2,3-dichloro-6-methylbenzoic acid and its salts;
2,4-dichlorophenoxyacetic acid and its salts and esters;
2,4,5-trichlorophenoxyacetic acid and its salts and esters;
2-methyl-4-chlorophenoxyacetic acid and its salts and esters;
2-(2,4,5-trichlorophenoxy)propionic acid and its salts and esters;
4-(2,4-dichlorophenoxy)butyric acid and its salts and esters;
4-(2-methyl-4-chlorophenoxy)butyric acid and its salts and esters;
2,3,6-trichlorophenylacetic acid and its salts;
3,6-endoxohexahydrophthalic acid and its salts;
dimethyl 2,3,5,6-tetrachloroterephthalate; trichloroacetic acid and its salts;
2,2-dichloropropionic acid and its salts;
2,3-dichloroisobutyric acid and its salts;
isopropylammonium 2-(4-isopropyl-4-methyl-5-oxo-2-imidazolin-2-yl)nicotinate;
2-[4,5-dihydro-4-methyl-4-(1-methylethyl)-5-oxo-1H-imidazol-2-yl]-3-quinolinecarboxylic acid;
6-(4-isopropyl-4-methyl-5-oxo-2-imidazolin-2-yl)-m-toluic acid, methyl ester and
6-(4-isopropyl-4-methyl-5-oxo-2-imidazolin-2-yl)-p-toluic acid, methyl ester;
N-(phosphomethyl)glycine isopropylammonium salt;
[3,5,6-trichloro-(2-pyridinyl)oxy]acetic acid;
3,7-dichloro-8-quinolinecarboxylic acid;
ammonium DL-homoalanin-4-yl(methyl)phosphinate;

### CARBAMIC ACID DERIVATIVES

ethyl N,N-di(*n*-propyl)thiolcarbamate;
*n*-propyl N,N-di(*n*-propyl)thiolcarbamate;
ethyl N-ethyl-N-(*n*-butyl)thiolcarbamate;
*n*-propyl N-ethyl-N-(*n*-butyl)thiolcarbamate;
2-chloroallyl N,N-diethyldithiocarbamate;
isopropyl N-phenylcarbamate;
isopropyl N-(*m*-chlorophenyl)carbamate;
4-chloro-2-butynyl-N-(*m*-chlorophenyl)carbamate;
methyl N-(3,4-dichlorophenyl)carbamate;
dinitro-*o*-(sec-butyl)phenol and its salts;
pentachlorophenol and its salts
S-(4-chlorobenzyl)-N,N-diethylthiolcarbamate;

### SUBSTITUTED UREAS

2-chloro-N-[(4-methoxy-6-methyl-1,3,5-triazin-2-yl)aminocarbonyl]-benzenesulfonamide;
3-(3,4-dichlorophenyl)-1,1-dimethylurea;
3-phenyl-1,1-dimethylurea;
3-(3,4-dichlorophenyl)-3-methoxy-1,1-dimethylurea;
3-(4-chlorophenyl)-3-methoxy-1,1-dimethylurea;
3-(3,4-dichlorophenyl)-1-n-butyl-1-methylurea;
3-(3,4-dichlorophenyl)-1-methoxy-1-methylurea;
3-(4-chlorophenyl)-1-methoxy-1-methylurea;
3-(3,4-dichlorophenyl)-1,1,3-trimethylurea;
3-(3,4-dichlorophenyl)diethylurea;
N-(4-isopropylphenyl)-N,N'-dimethylurea;
dichloral urea;
methyl 2-[[[[(4,6-dimethyl-2-pyrimidinyl)amino]-carbonyl]amino]sulfonyl]benzoate;
N-((6-methoxy-4-methyl-1,3,5-triazin-2-yl)aminocarbonyl)-2-(2-chloroethoxy)-benzenesulfonamide;
2-[[[(4-chloro-6-methoxypyrimidine-2-yl)aminocarbonyl]amino]-sulfonyl]benzoic acid, ethyl ester;
methyl 2-[[[[(4-methoxy-6-methyl-1,3,5-triazin-2-yl)amino]-carbonyl]amino]-sulfonyl]benzoate;
methyl 3-[[(4-methoxy-6-methyl-1,3,5-triazin-2-yl)aminocarbonyl]aminosulfonyl]-2-thio-phenecarboxylate;
methyl 2-[[[[[(4,6-dimethoxypyrimidin-2-yl)amino]carbonyl]amino]sulfonyl]methyl]benzoate;
methyl 2-[[[[(4-methoxy-6-methyl-1,3,5-triazin-2-yl)methylamino]carbonyl]amino]sulfonyl]benzoate;

### SUBSTITUTED TRIAZINES

2-chloro-4,6-bis(ethylamino)-s-triazine;
2-chloro-4-ethylamino-6-isopropylamino-s-triazine;
2-chloro-4,6-bis(3-methoxy-n-propylamino)-s-triazine;
2-methoxy-4,6-bis(isopropylamino)-s-triazine;
2-chloro-4-ethylamino-6-(3-methoxy-*n*-propylamino)-s-triazine;
2-methylmercapto-4,6-bis(isopropylamino)-s-triazine;
2-methylmercapto-4,6-bis(ethylamino)-2-triazine;
2-methylmercapto-4-ethylamino-6-isopropylamino-s-triazine;
2-chloro-4,6-bis(isopropylamino)-s-triazine;
2-methoxy-4-ethylamino-6-isopropylamino-s-triazine;
2-methylmercapto-4-(2-methoxyethylamino)-6-isopropylamino-s-triazine;
4-amino-6-(t-butyl)-3-(methylthio)-1,2,4-triazine-5(4H)-one;

### DIPHENYL ETHER DERIVATIVES

2,4-dichloro-4'-nitrodiphenyl ether;
2,4,6-trichloro-4'-nitrodiphenyl ether;
2,4-dichloro-6-fluoro-4'-nitrodiphenyl ether;
3-methyl-4'-nitrodiphenyl ether;
3,5-dimethyl-5'-nitrodiphenyl ether;
2,4'-dinitro-4-(trifluoromethyl)diphenyl ether;
2,4-dichloro-3'-methoxy-4'-nitrodiphenyl ether;
sodium 5-(2-chloro-4-(trifluoromethyl)phenoxy)-2-nitrobenzoate;
2-chloro-1-(3-ethoxy-4-nitrophenoxy)-4-(trifluoromethyl)benzene;
1-(carboethoxy)ethyl 5-[2-chloro-4-(trifluoromethyl)-phenoxy]-2-nitrobenzoate;
5-[2-chloro-4-(trifiuoromethyl)phenoxyl]-N-(methylsulphony)-2-nitrobenzamide;

### ANILIDES

2-chloro-N-(2-ethyl-6-methylphenyl)-N-(2-methoxy-1-methylethyl)acetamide;
2-chloro-2',6'-diethyl-N-(2-propyloxyethyl)acetanilide;
N-(3,4-dichlorophenyl)propionamide;
N-(3,4-dichlorophenyl)methacrylamide;
N-(3-chloro-4-methylphenyl)-2-methylpentanamide;
N-(3,4-dichlorophenyl)trimethylacetamide;
N-(3,4-dichlorophenyl)-alpha,alpha-dimethylvaleramide;
N-isopropyl-N-phenylchloroacetamide;
N-n-butoxymethyl-N-(2,6-diethylphenyl)chloroacetamide;
N-methoxymethyl-N-(2,6-diethylphenyl)chloroacetamide;

### OXYPHENOXY HERBICIDES

2-(4-(2,4-dichlorophenoxy)phenoxy)methyl propionate;
methyl 2-(4-(3-chloro-5-(trifluoromethyl)-2-pyridinyloxy)phenoxy)propanoate;
butyl (R)-2-[4-[5-(trifluoromethyl)-2-pyridinyloxy]-phenoxy]propionate;
ethyl 2-[4-[(6-chloro-2-benzoxazolyl)oxy]phenoxy]propanoate;
butyl 2-[4-[[5-(trifluoromethyl)-2-pyridinyl]oxy]phenoxy]propionate;
2-[4-[(6-chloro-2-quinoxalinyl)oxy]phenoxy]propionic acid, ethyl ester;

### URACILS

5-bromo-3-s-butyl-6-methyluracil;
5-bromo-3-cyclohexyl-1,6-dimethyluracil;
3-cyclohexyl-5,6-trimethyleneuracil;
5-bromo-3-isopropyl-6-methyluracil;
3-tert-butyl-5-chloro-6-methyluracil;

### NITRILES

2,6-dichlorobenzonitrile;
diphenylacetonitrile;
3,5-dibromo-4-hydroxybenzonitrile;
3,5-diiodo-4-hydroxybenzonitrile;

### OTHER ORGANIC HERBICIDES

2-chloro-N,N-diallylacetamide;
N-(1,1-dimethyl-2-propynyl)-3,5-dichlorobenzamide;
maleic hydrazide;
3-amino-1,2,4-triazole; monosodium methanearsonate;
disodium methanearsonate;
N,N-dimethyl-alpha,alpha-diphenylacetamide;
N-N-di(n-propyl)-2,6-dinitro-4-(trifluoromethyl)aniline;
N,N-di(n-propyl)-2,6-dinitro-4-methylaniline;
N,N-di(n-propyl)-2,6-dinitro-4-methylsulfonylaniline;
O-(2,4-dichlorophenyl)-O-methyl isopropylphosphoramidothioate;
4-amino-3,5,6-trichloropicolinic acid;
2,3-dichloro-1,4-naphthoquinone;
di(methoxythiocarbonyl)disulfide;
3-(1-methylethyl)-1H-2,1,3-benzothiadiazin-(4)3H-one-2,2-dioxide;
6,7-dihydrodipyridol[1,2-a:2',1'-c]pyrazidiium salts;
1,1'-dimethyl-4,4'-bipyridinium salts;
3,4,5,6-tetrahydro-3,5-dimethyl-2-thio-2H-1,3,5-thiadiazine;
2-[1-(ethoxyimino)butyl]-5-[s-(ethylthio)propyl]-3-hydroxy-2-cyclohexen-1-one;
2-(2-chlorophenyl)methyl-4,4-dimethyl-3-isoxazolidinone;
N-(1-ethylpropyl)-3,4-dimethyl-2,6-dinitrobenzamide;
4-chloro-5-(methylamino)-2-(a,a,a-trifluoro-*m*-toluyl)-3-(2H)-pyridazinone;
2-(3,5-dichlorophenyl)-2-(2,2,2-trichloromethyl)oxirane.

When mixtures of herbicides are employed, the relative proportions which are used will depend upon the crop to be treated and the degree of selectivity in weed control desired. The herbicidal activity of the 2-arylpyrimidines of the present invention towards a number of common weeds was evaluated using a greenhouse method of testing. Using the procedures described below, the aryl pyrimidines of the present invention were evaluated for control of weeds selected from the following:

| Monocots | |
|---|---|
| Barnyardgrass (BYG) | Echinochloa crus-galli |
| Crabgrass (CRB) | Digitaria sanguinilis |
| Foxtail (FOX) | Setaria viridis |
| Johnsongrass (JON) | Sorghum halepense |
| Meadow Foxtail (MF) | Alopecurus pratensis |
| Nutsedge (NUT) | Cyperus esculentus |
| Wild Oat (WO) | Avena fatua |

| Dicots | |
|---|---|
| Beggartick (BID) | Bidens pilosa |
| Cocklebur (CKL) | Xanthium strumarium |
| Morningglory (MG) | Ipomoea lacunosa |
| Nightshade (NS) | Solanum nigrum |
| Pigweed (PIG) | Amaranthus retroflexus |
| Smartweed (SMT) | Polygonum lapathifolium |
| Velvetleaf (VEL) | Abutilon theophrasti |

The following test procedure was employed. Seeds of selected plants were planted in flats or pots. For preemergence tests, immediately after planting, the test compound was sprayed directly onto the soil surface. The flats or pots were placed in the greenhouse and then watered. For postemergence tests, the seeds were allowed to germinate and grow for 10 to 21 days. Before application, each series of test plants were selected for uniformity, size and stage of development. The test plants were then treated with the test compound, returned to the greenhouse and watered.

The compound to be evaluated was dissolved in an appropriate solvent, usually acetone, and sprayed over the flats or pots using a carrier volume equivalent to 25 or 50 gallons per acre at the rate of application in pounds per acre (Lb/A) or grams per hectare (g/Ha) specified in the below tables. About two or three weeks after application of the test compound, the state of growth of the plant was observed. Each species was evaluated on a scale of 0-100 in which 0 equals no activity and 100 equals total control. The column heading abbreviations in the below tables for the plants tested are the same as for the monocots and dicots hereinabove. The dash ("-") entry signifies no testing for the specified conditions.

**TABLE 3**

| EX. | TYPE | lb/a | CKL | MG | PIG | SMT | VEL | BYG | FOX | JON | NUT | WO |
|---|---|---|---|---|---|---|---|---|---|---|---|---|
| 1 | PRE | 4.00 | 20 | 100 | 100 | 100 | 100 | 100 | 100 | 99 | 55 | 100 |
| | POST | 4.00 | 25 | 70 | 100 | 100 | 55 | 80 | 50 | 0 | 15 | 100 |
| 2 | PRE | 4.00 | 5 | 100 | 100 | 100 | 100 | 100 | 100 | 100 | 65 | 60 |
| | POST | 4.00 | 15 | 75 | 75 | 50 | 35 | 80 | 75 | 0 | 0 | 35 |
| 4 | PRE | 4.00 | 25 | 100 | 100 | 100 | 100 | 100 | 100 | 100 | 85 | 100 |
| | POST | 4.00 | 20 | 45 | 100 | 55 | 50 | 95 | 95 | 70 | 35 | 90 |
| 5 | PRE | 4.00 | 40 | 100 | 100 | 100 | 100 | 100 | 100 | 100 | 100 | 98 |
| | POST | 4.00 | 20 | 85 | 70 | 99 | 65 | 98 | 95 | 65 | 80 | 70 |
| 6 | PRE | 4.00 | 15 | 100 | 100 | 100 | 100 | 100 | 100 | 100 | 100 | 85 |
| | POST | 4.00 | 0 | 10 | 45 | 40 | 0 | 20 | 0 | 70 | 15 | 0 |
| 7 | PRE | 4.00 | 0 | 10 | - | 100 | 60 | 90 | 100 | 55 | - | 40 |
| | POST | 4.00 | 0 | 0 | 20 | 15 | 20 | 0 | 0 | 0 | 0 | 0 |
| 221 | PRE | 4.00 | 15 | 100 | 100 | 100 | 100 | 100 | 100 | 100 | 80 | 98 |
| | POST | 4.00 | 15 | 35 | 20 | 60 | 0 | 85 | 98 | 40 | 15 | 20 |
| "-" EQUALS NOT TESTED | | | | | | | | | | | | |

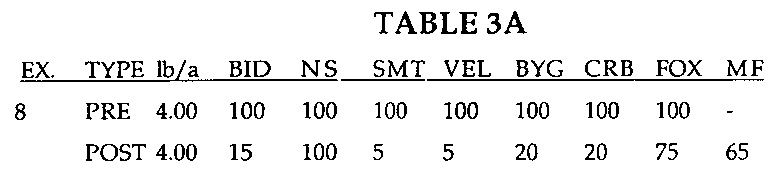

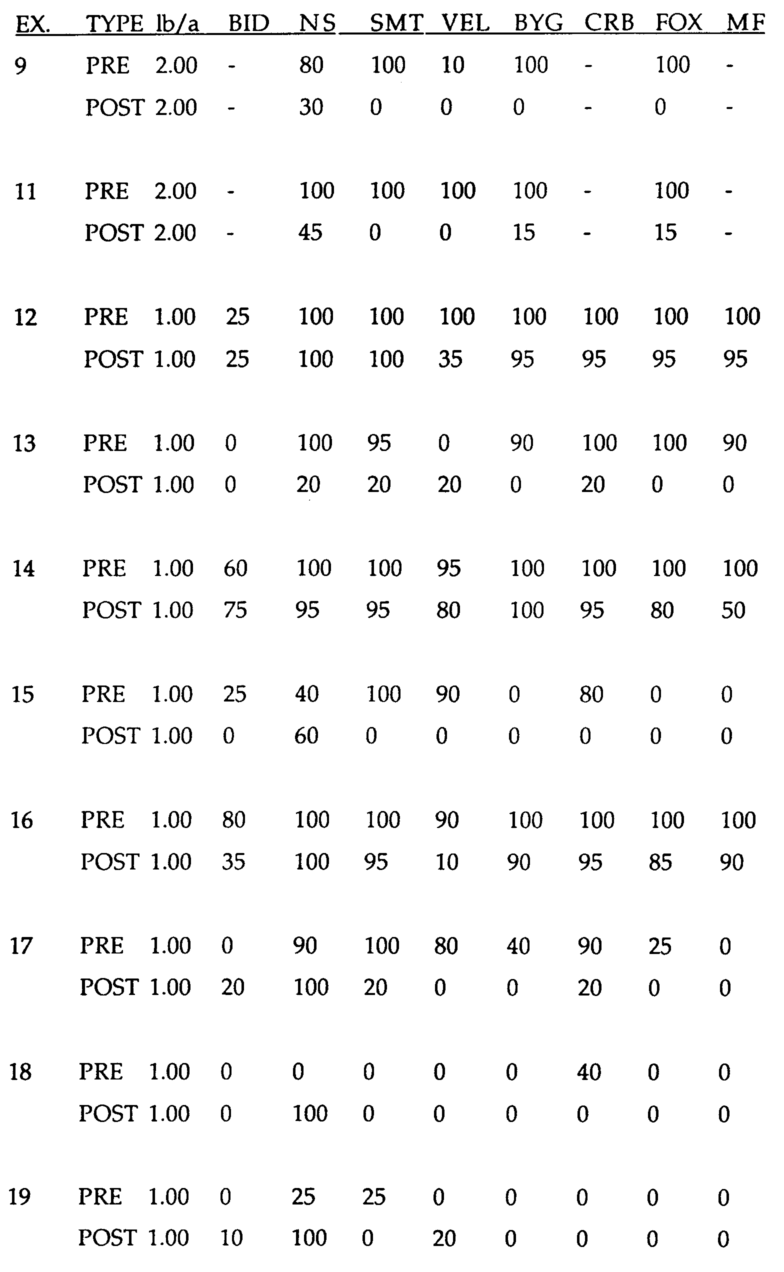

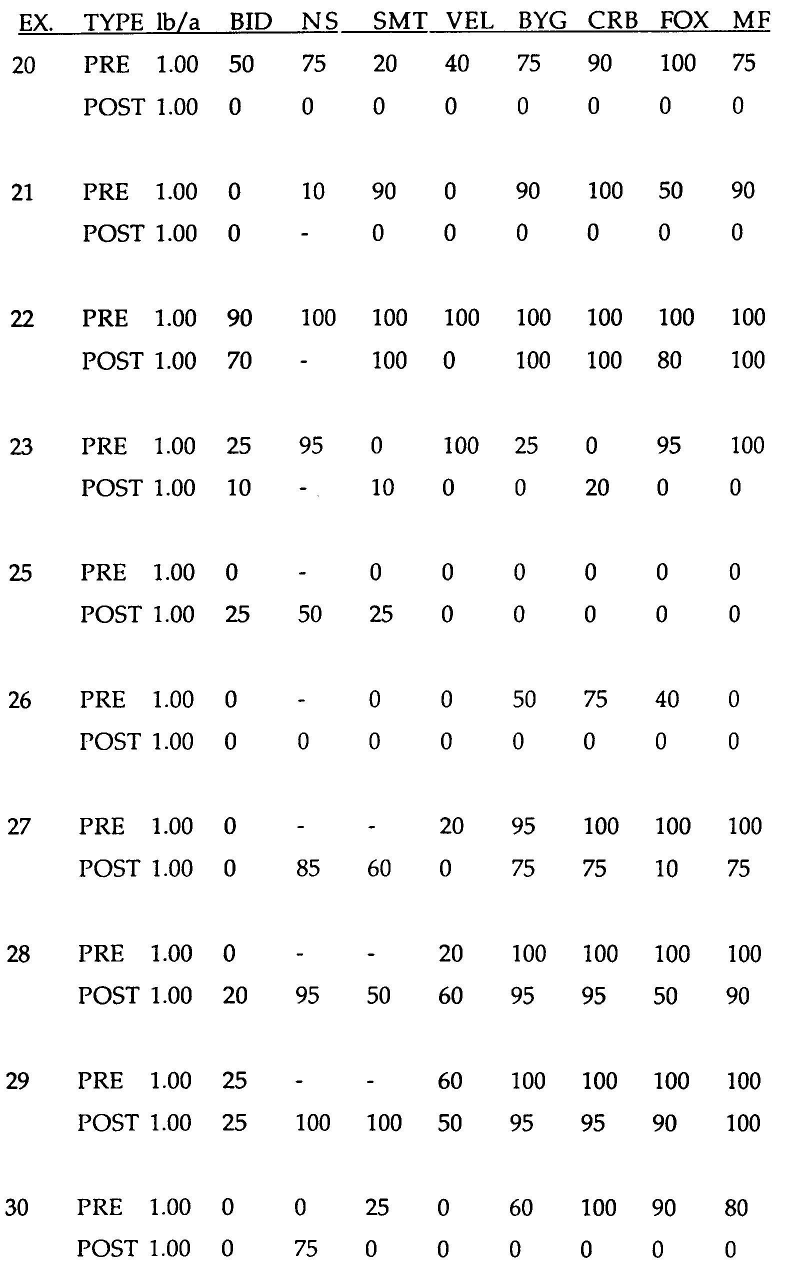

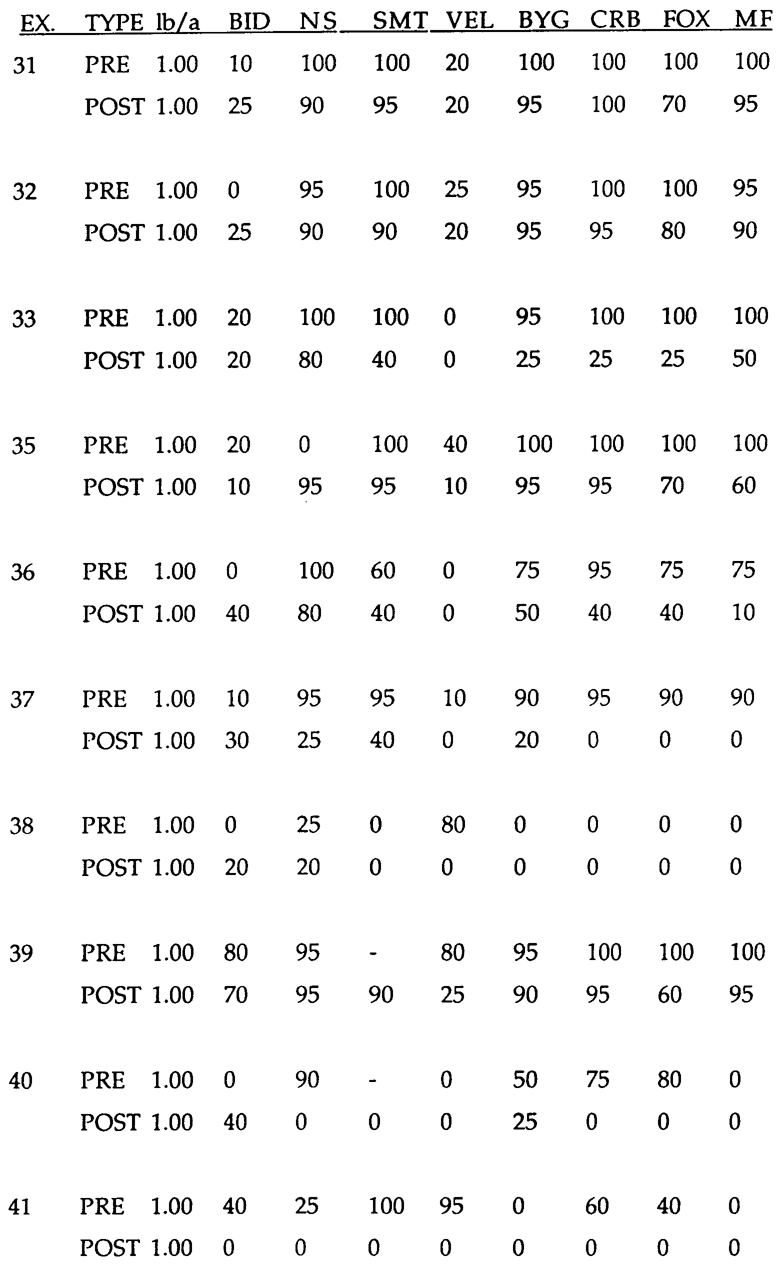

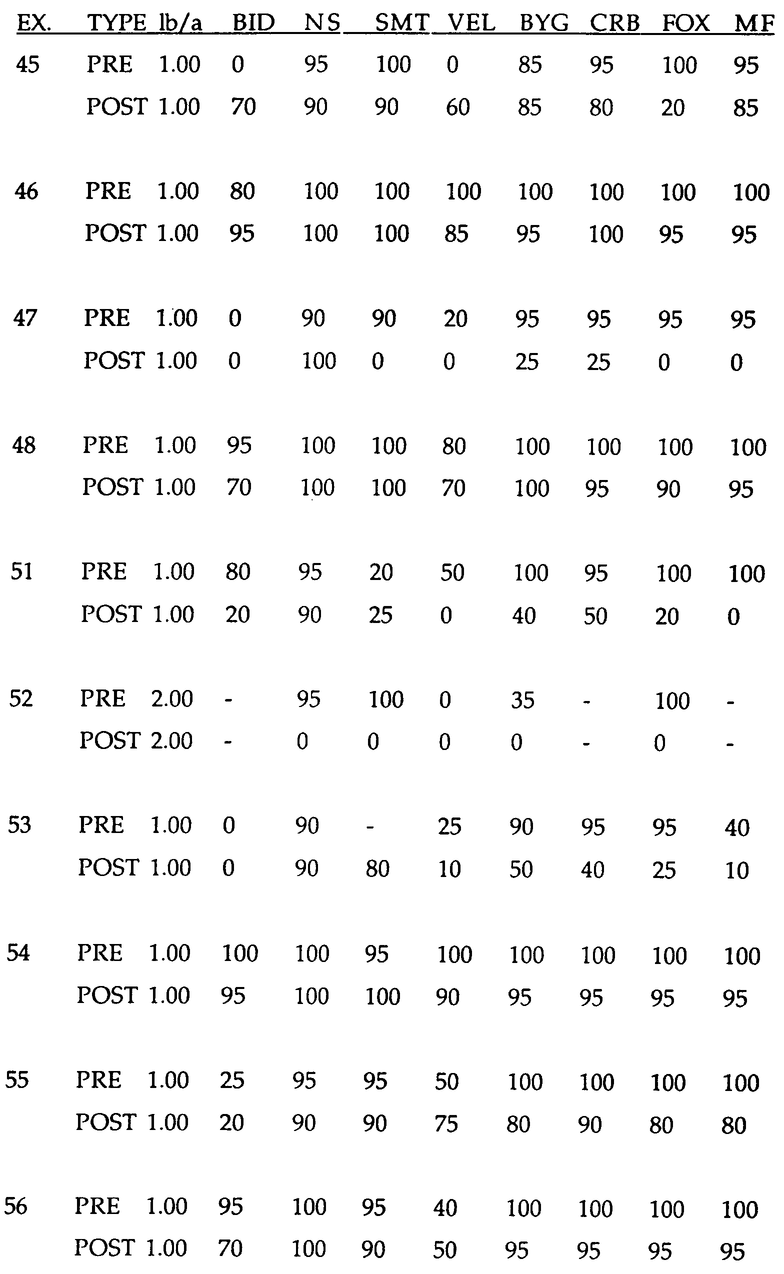

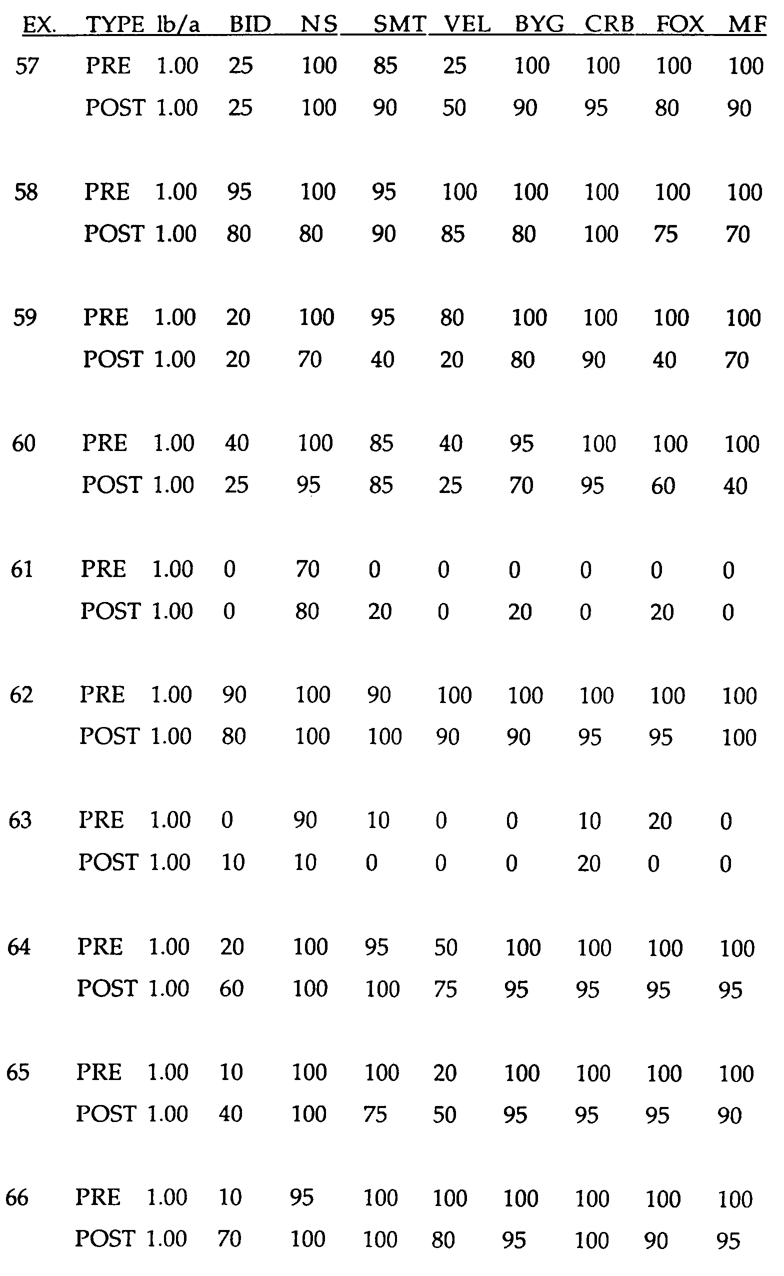

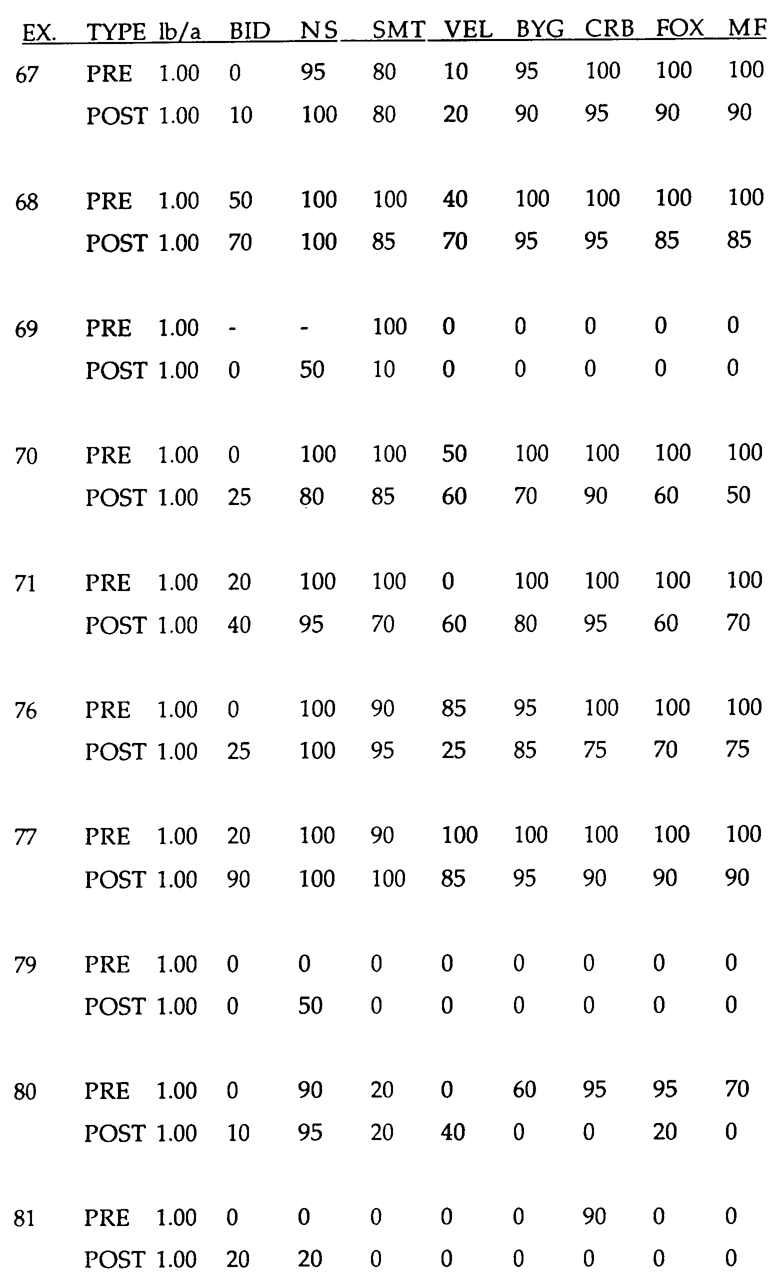

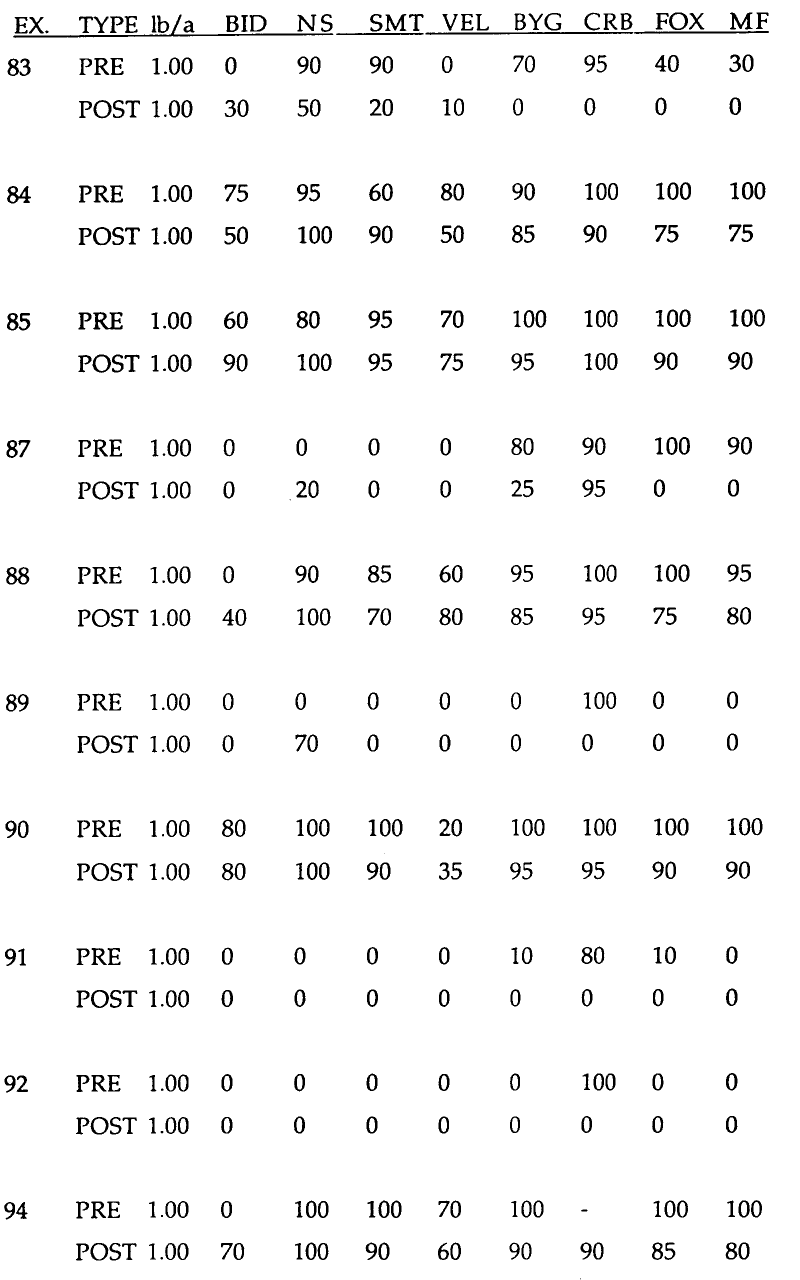

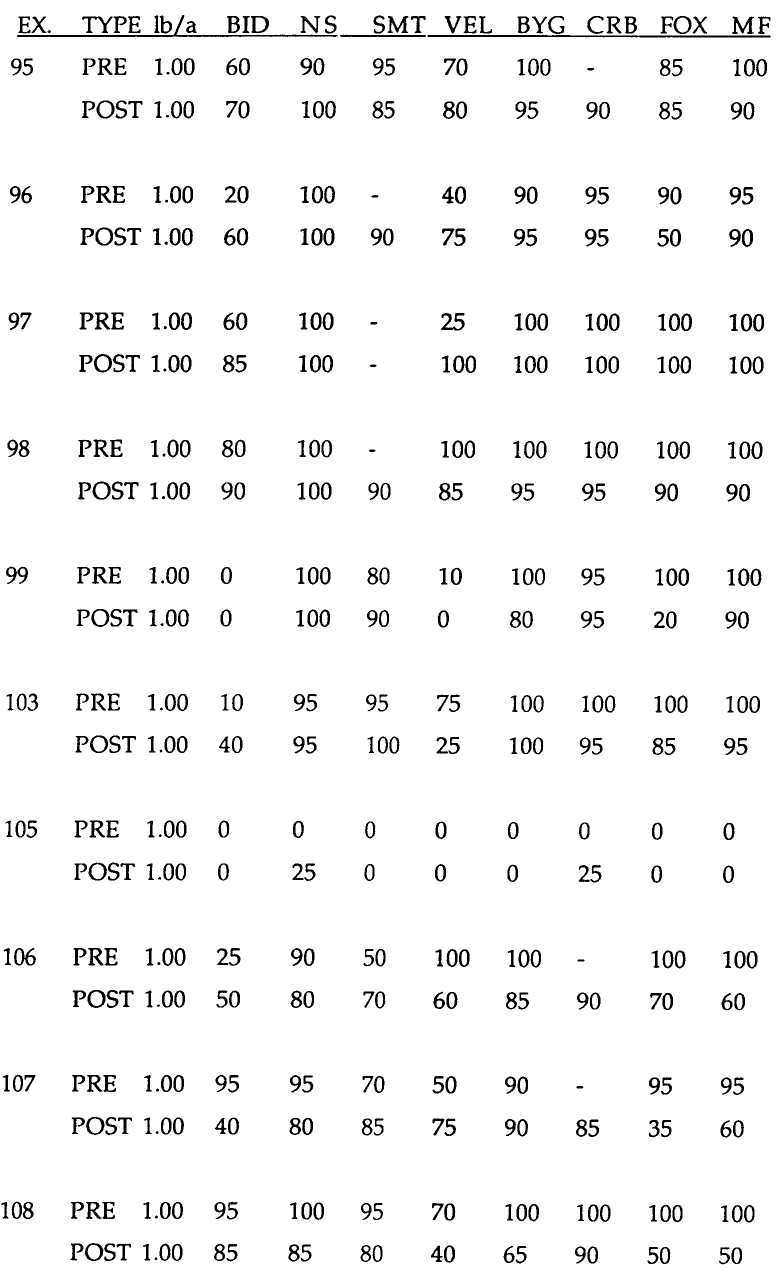

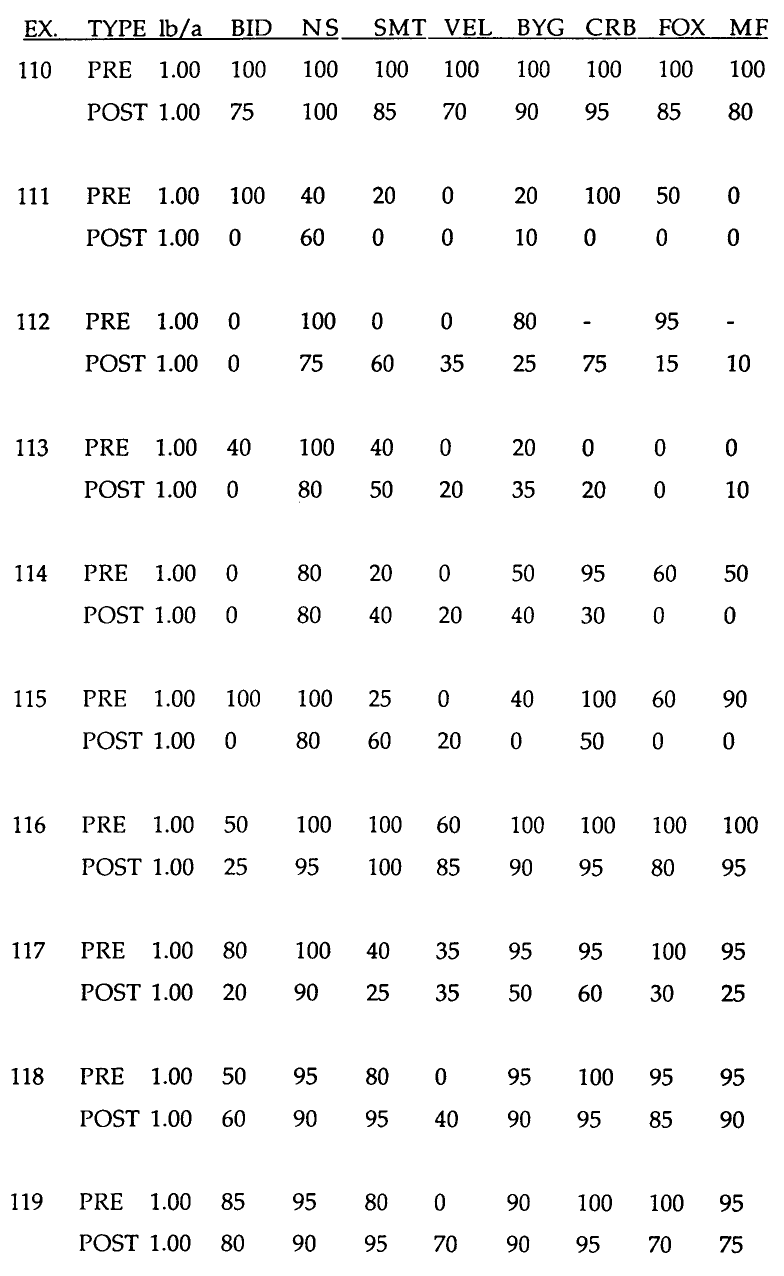

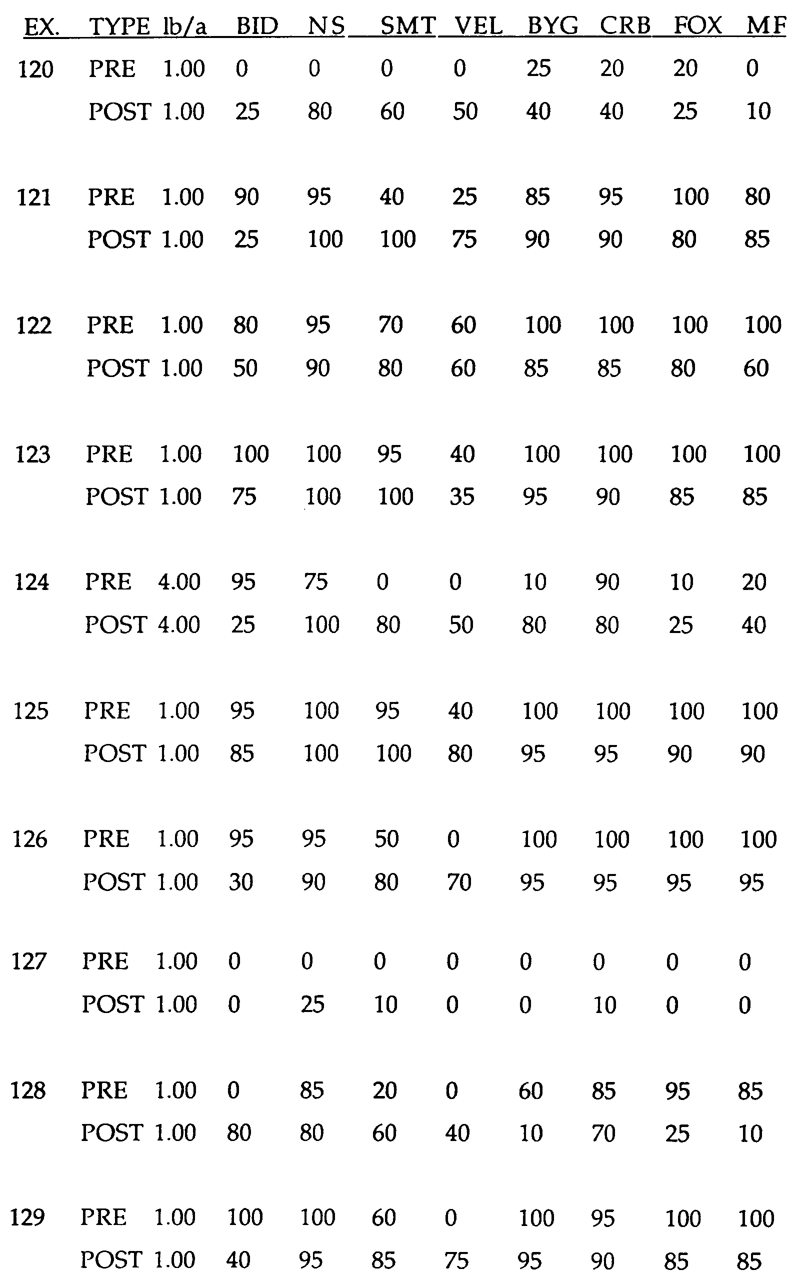

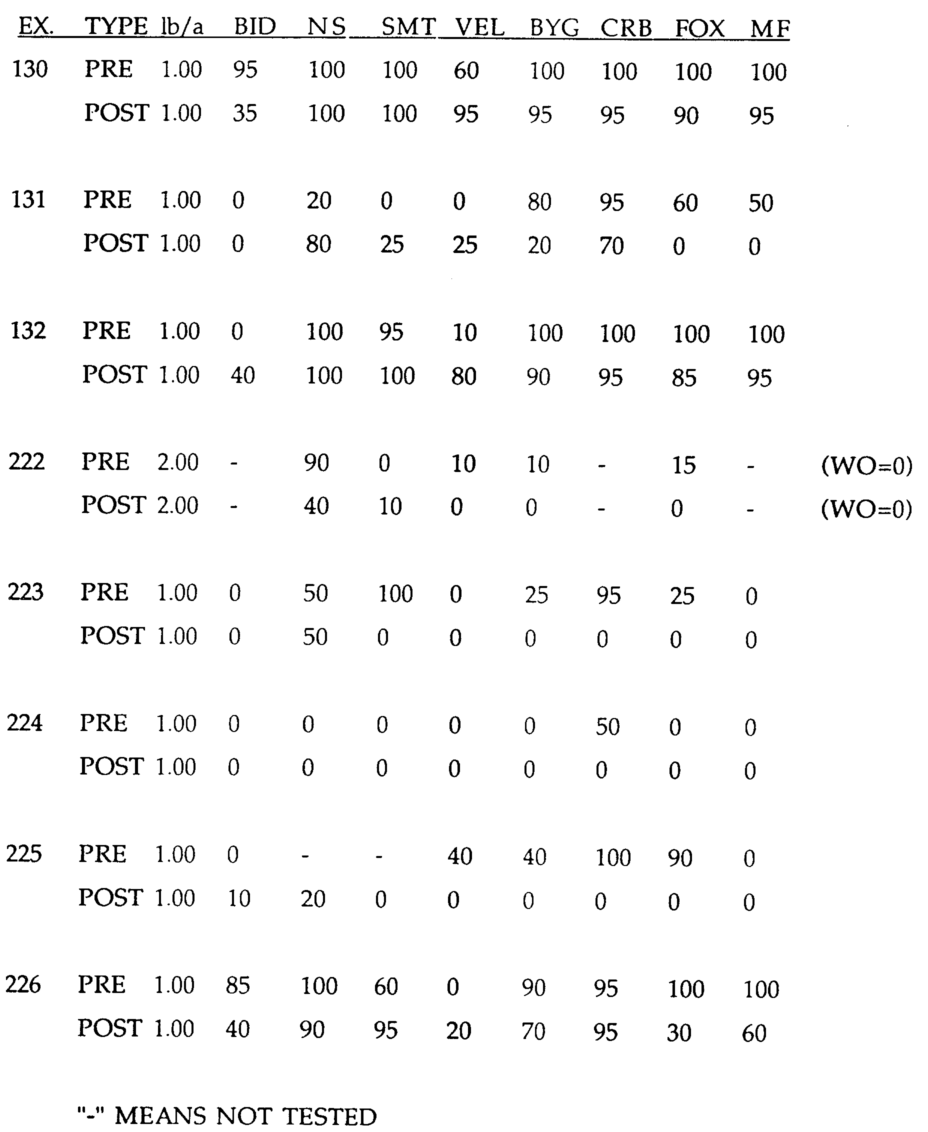

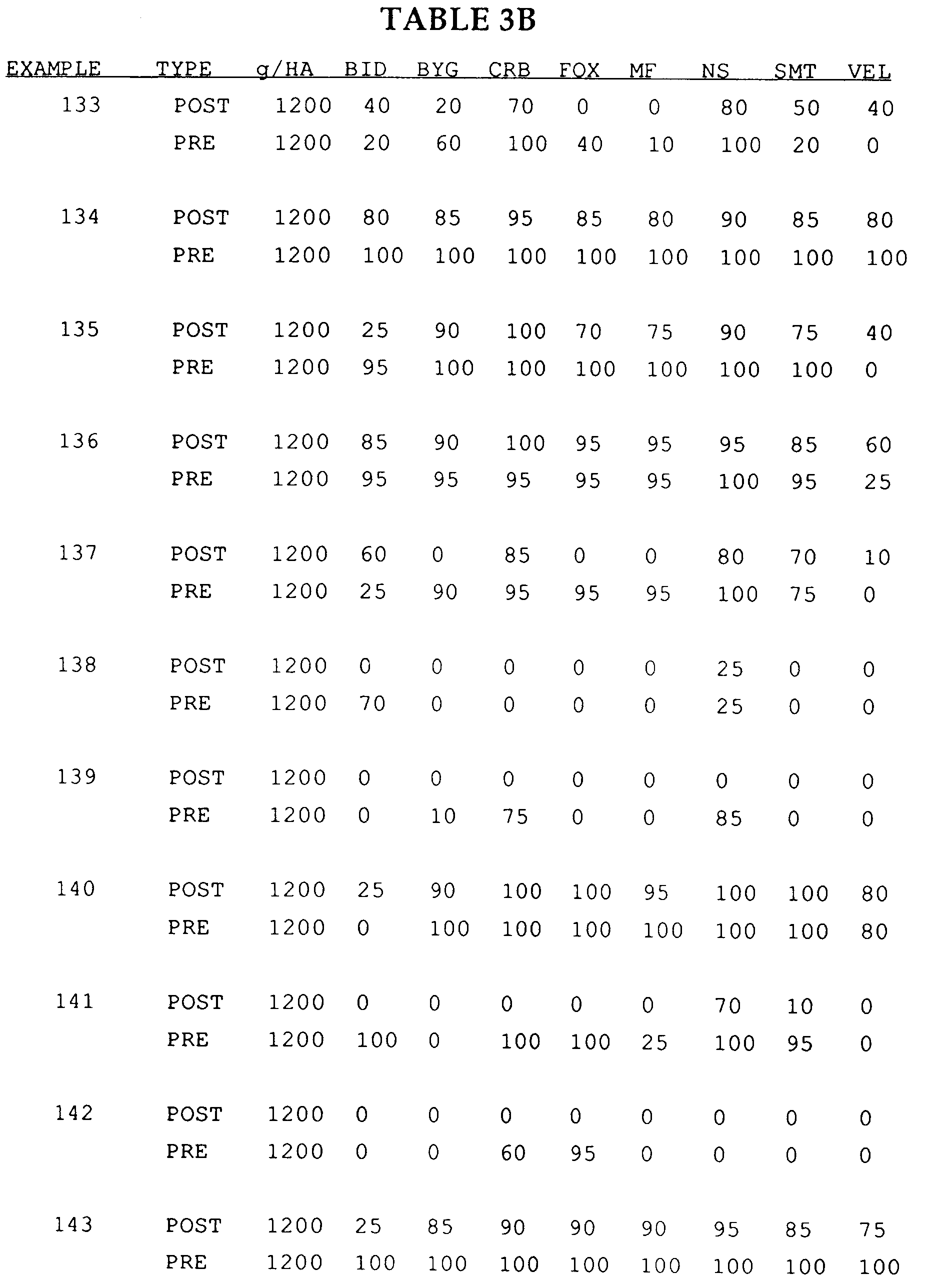

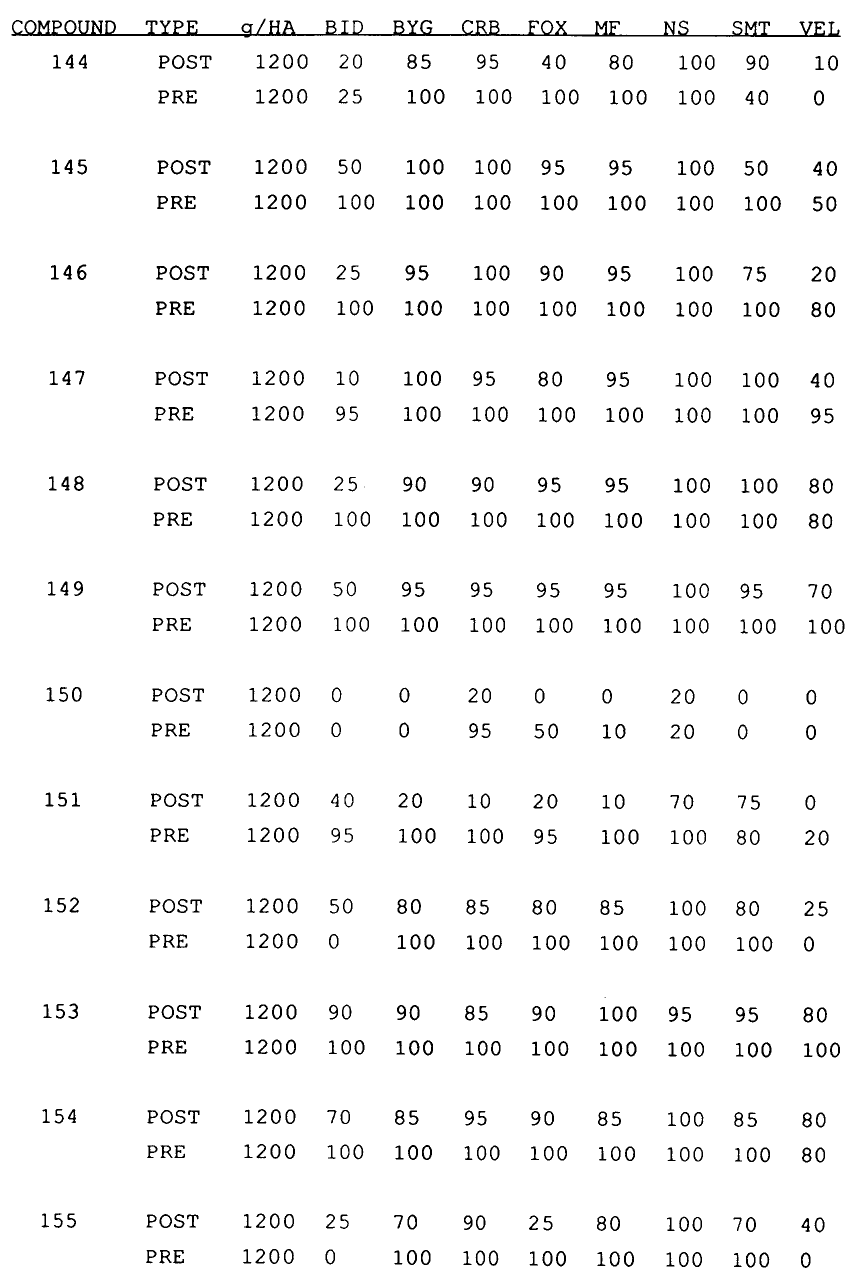

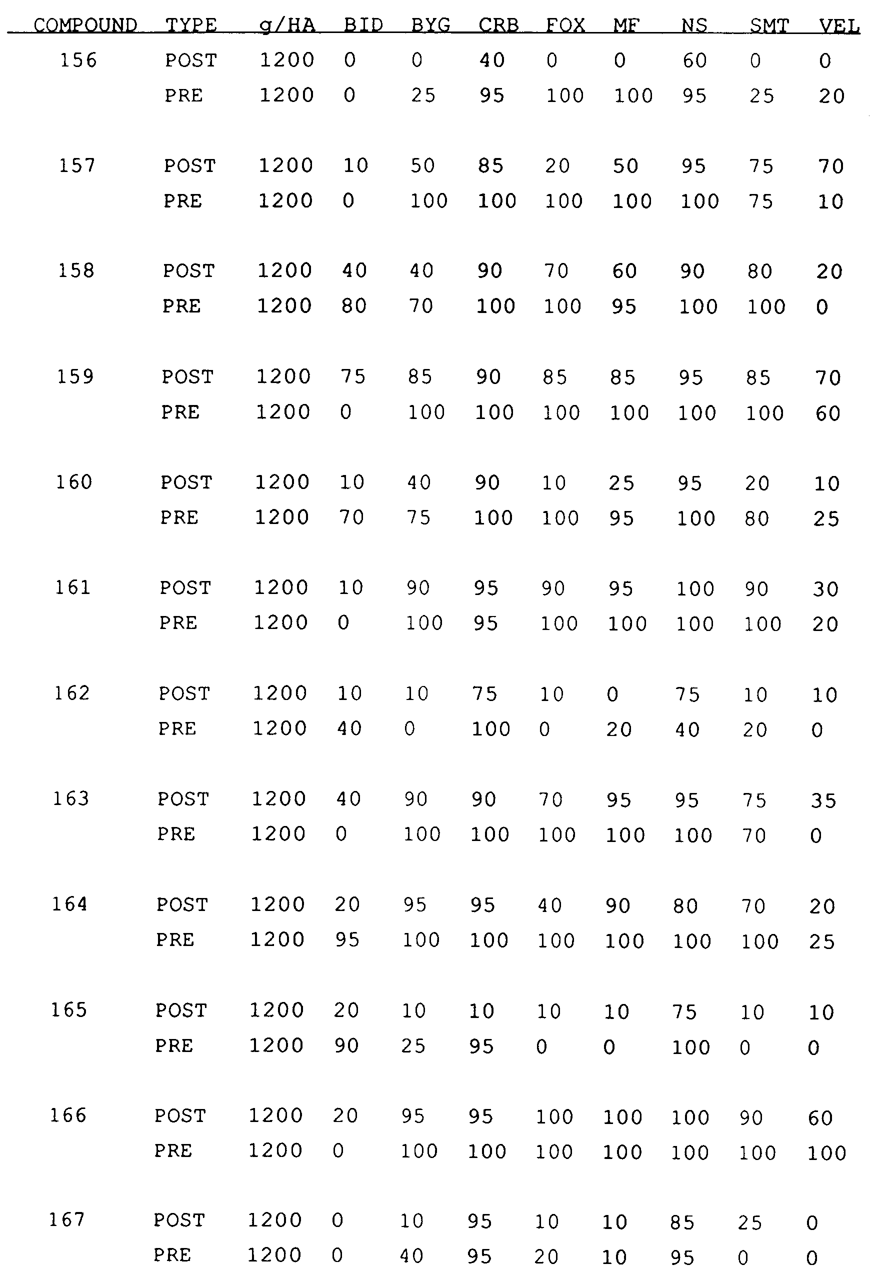

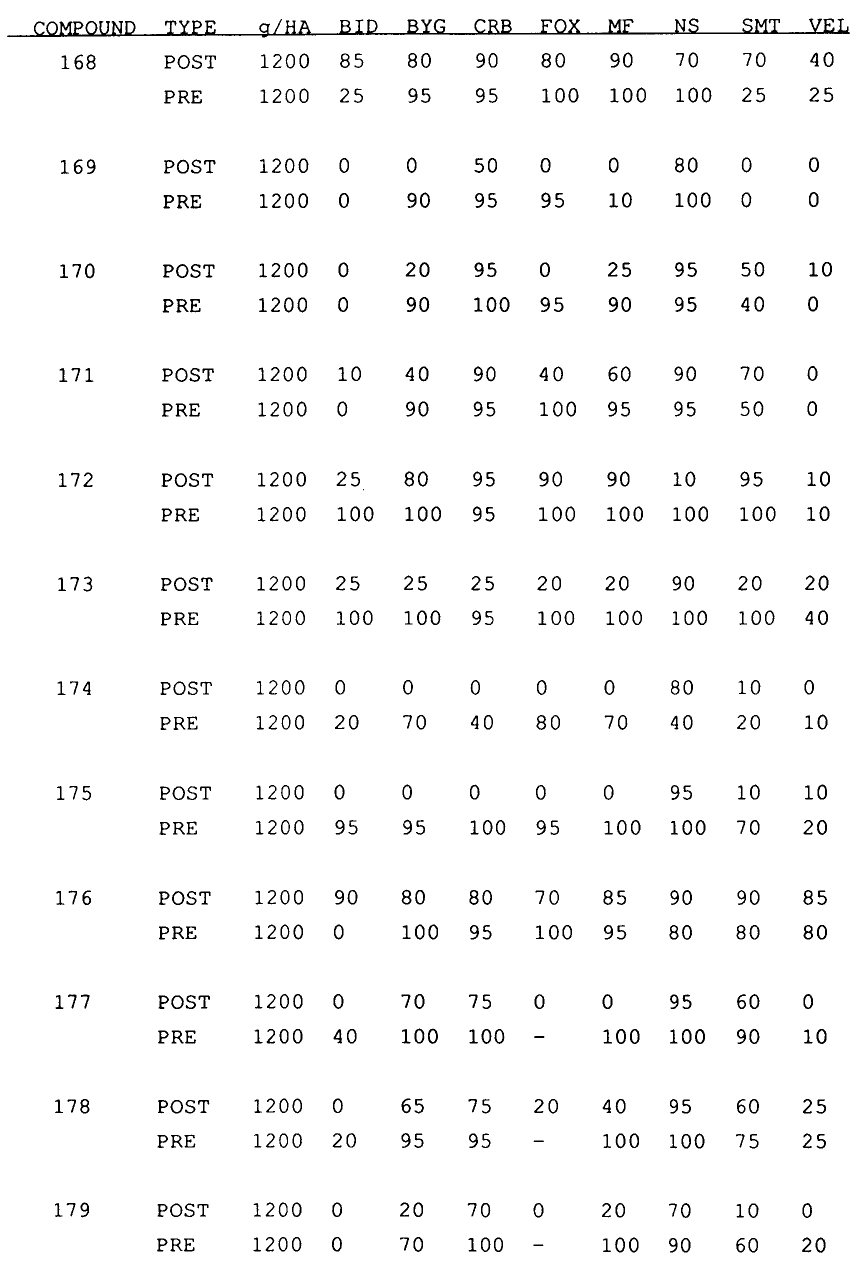

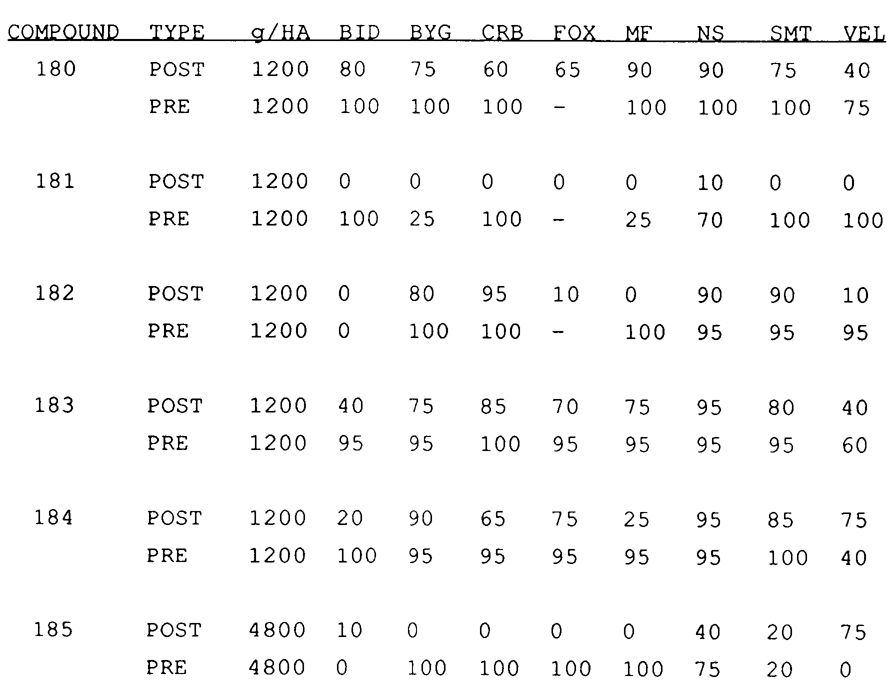

## Claims

1. A compound of the formula: 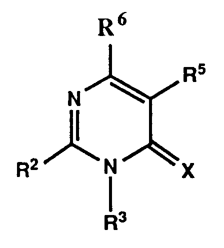 wherein
(a) R² is a furyl, phenyl, naphthyl, pyridyl, or thienyl group,
each of said groups is optionally substituted with up to three substituents independently selected from a bromo, chloro, fluoro, (C₁-C₁₂)alkyl, cyclo(C₃-C₈)alkyl, (C₂-C₁₂)alkenyl, cyclo(C₃-C₈)alkenyl, (C₁-C₁₂)alkynyl, halo(C₁-C₁₂)alkyl, polyhalo(C₁-C₁₂)alkyl, halo(C₁-C₁₂)alkenyl, polyhalo(C₂-C₁₂)alkenyl, halo(C₂-C₆)alkynyl, polyhalo(C₂-C₆)alkynyl, (C₁-C₁₂)alkoxy, (C₁-C₁₂)alkylthio, (C₁-C₁₂)alkylsulfonyl, (C₁-C₁₂)alkylsulfinyl, phenyl, phen(C₁-C₁₂)alkyl, phen(C₂-C₁₂)alkenyl, phen(C₁-C₁₂)alkynyl, cyano, halo(C₁-C₁₂)alkoxy, carbo(C₁-C₆)alkoxy, 1,3-dioxolan-2-yl, hydroxyimino, or nitro group; and when R² is pyridyl, such pyridyl group is optionally substituted with oxygen on the nitrogen of the pyridyl group; or R² is a furyl, phenyl, naphthyl, pyridyl or thienyl group having a fused ring moiety composed of an oxymethyleneoxy or an oxoethyleneoxy link bonded to adjacent carbon atoms of said group;
(b) R³ is a (C₁-C₃)alkyl, halo(C₁-C₂)alkyl, polyhalo (C₁-C₃)alkyl, (C₃-C₄)alkenyl, (C₅-C₆)alkenynyl, (C₃-C₆)alkynyl, (C₁-C₆)alkoxy(C₁-C₆)alkyl, di(C₁-C₆)alkoxy(C₁-C₆)alkyl, halo(C₁-C₆)alkoxy(C₁-C₆)alkyl, 2-oxo(C₂-C₃)alkyl, trimethylsilyl(C₃-C₄)alkynyl or cyano(C₁-C₆)alkyl group, each of said (C₃-C₄)alkenyl, or (C₃-C₆)alkynyl group is optionally substituted with up to five halogens; and
(c) R⁵ and R⁶ are joined together to form a fused ring moiety bonded to the pyrimidine ring at the 5 and 6 positions and containing two to five atoms in its link, each of said atoms being independently carbon, oxygen or sulfur of which the carbon atoms are optionally substituted with (C₁-C₃)alkyl, polyhalo(C₁-C₃)alkyl or halo groups; and
(d) X is oxygen or sulfur.

2. A compound as claimed in claim 1, wherein R² is
(a) phenyl, 3-methylphenyl, 3-methoxyphenyl, 3-nitrophenyl, 4-fluorophenyl, 4-chlorophenyl, 3-trifluoromethylphenyl, 3-bromophenyl, 3-chlorophenyl, 3-fluorophenyl, 3-trifluoromethoxyphenyl, 3-cyanophenyl, 3-(1,3-dioxolan-2-yl)-phenyl, 3-(hydroxyimino)phenyl, 2-fluorophenyl, 2-chlorophenyl, 2-trifluoromethoxyphenyl, 3,5-difluorophenyl, 3,5-dichlorophenyl, 2,4-difluorophenyl, 2,5-difluorophenyl, 3-chloro-4-fluorophenyl, 3,4-difluorophenyl, 3-fluoro-5-trifluoromethylphenyl, or 3,4,5-trifluorophenyl; or
(b) 6-chloro-2-pyridyl, 3-pyridyl, 1-methyl-3-pyridinium halide, 5-bromo-3-pyridyl, 5,6-dichloro-3-pyridyl, 5-chloro-3-pyridyl, 1-oxo-3-pyridyl, 4-pyridyl, 2-fluoro-4-pyridyl, 2-methyl-4-pyridyl, 2-methoxy-4-pyridyl, 2-cyano-4-pyridyl, 1-oxo-4-pyridyl, 2-chloro-4-pyridyl, 2-chloro-6-methyl-4-pyridyl, 2,6-difluoro-4-pyridyl or 2,6-dichloro-4-pyridyl; or
(c) 2-furyl or 3-furyl; or
(d) 2-thienyl, 3-thienyl, 4-chloro-2-thienyl, 5-chloro-2-thienyl, 5-chloro-3-thienyl, or 2,5-dichloro-3-thienyl.

3. A compound as claimed in claim 1 or claim 2, wherein R³ is allyl, pent-2-ynyl, prop-2-ynyl, but-2-ynyl, methoxymethyl, 2-methoxyethyl, acetonyl, 2,2-dimethoxypropyl, pent-4-en-2-ynyl, 3-chloroallyl, 3-iodopropargyl, 3-trimethylsilyl propargyl, or cyanomethyl.

4. A compound as claimed in any preceding claim, wherein R⁵ and R⁶ are joined together to form a two to five membered link group containing from one to five substituted or unsubstituted methylene (-CH₂-) groups and optionally one oxygen or sulfur atom, wherein said methylene carbons may be substituted with a (C₁-C₃)alkyl, polyhalo(C₁-C₃)alkyl or halo groups.

5. A compound as claimed in any preceding claim, wherein X is oxygen.

6. A compound as claimed in claim 4, wherein the -R⁵--R⁶- linkage is -CH=CH-CH=CH-, -CH=CH-S-, -S-CH=CH-, -O-CH=CH-, -CH=CH-O- or -(CH₂)_{y}-where y is 1 to 4, the carbon atoms being optionally substituted with (C₁-C₃)alkyl, monohalo(C₁-C₃)alkyl, polyhalo(C₁-C₃)alkyl or halo.

7. A compound as claimed in claim 10, wherein R is 2-furyl, phenyl, 3-fluorophenyl, 3-chlorophenyl, 3,5-difluorophenyl, 3,5-dichlorophenyl, 2-chloro-4-pyridyl, 2-fluoro-4-pyridyl or 2,6-dichloro-4-pyridyl.

8. A compound as claimed in any preceding claim, which is 2-phenyl-3-propargyl-3,5,6,7-tetrahydro-4H-cyclopentapyrimidin-4-one; or 2-phenyl-3-propargyl-3,5,6,7,8-pentahydro-4H-cyclohexapyrimidin-4-one.

9. A herbicidal composition comprising a herbicidally effective amount of a compound as defined in any preceding claim, and an agronomically acceptable carrier.

10. A method of controlling a weed comprising applying a herbicidally effective amount of a composition or compound as defined in any preceding claim to said weed or to the locus of said weed or to the growth medium of said weed.

11. The use of a compound or composition as defined in any of claims 1 to 9 as a herbicide.
